# EUROPÄISCHE PATENTSCHRIFT

(11) **EP 3 485 274 B1**
(45) Veröffentlichungstag und Bekanntmachung des Hinweises auf die Patenterteilung: **24.04.2024**
(21) Anmeldenummer: 17751253.0
(22) Anmeldetag: 11.07.2017
(51) Int. Cl.: G01N 33/36, D06F 39/00

(54) **VERFAHREN ZUR ERMITTLUNG VON BEHANDLUNGSPARAMETERN EINER TEXTILIE ÜBER STRUKTURINFORMATION**
METHOD FOR ASCERTAINING TREATMENT PARAMETERS OF A TEXTILE BY MEANS OF STRUCTURAL INFORMATION
PROCÉDÉ POUR DÉTERMINER DES PARAMÈTRES DE TRAITEMENT D'UN TEXTILE GRÂCE À DES INFORMATIONS DE STRUCTURE

(30) Priorität: 15.07.2016 DE 102016212979; 12.06.2017 DE 102017209865
(43) Veröffentlichungstag der Anmeldung: 22.05.2019
(73) Patentinhaber: Henkel AG & Co. KGaA, 40589 Düsseldorf (DE)
(72) Erfinder: KESSLER, Arnd, 40789 Monheim am Rhein (DE); NITSCH, Christian, 40591 Düsseldorf (DE); ZÜCHNER, Lars, 40764 Langenfeld (DE); WAWER, Georg, 1040 Wien (AT); MÜLLER, Alexander, 40789 Monheim (DE)
(86) Internationale Anmeldenummer: PCT/EP2017/067337
(87) Internationale Veröffentlichungsnummer: WO 2018/011173

(56) Entgegenhaltungen:
- WO-A1-01/46509
- WO-A1-2004/059075
- DE-A1- 19 855 503
- DE-A1-102011 087 274
- DE-A1-102013 210 996
- JP-A- S59 178 306
- US-A1- 2007 248 246
- US-A1- 2008 078 035
- US-A1- 2010 205 823

## Beschreibung

### Gebiet der Erfindung

Die Erfindung betrifft Verfahren und Vorrichtungen, mit welchen über ein zerstörungsfreies Bestimmen von Strukturinformation indikativ für zumindest einen Teil der Struktur einer Textilie mindestens ein Behandlungsparameter der Textilie zumindest teilweise basierend auf der Strukturinformation ermittelt wird.

### Hintergrund der Erfindung

Textilien müssen bei Gebrauch regelmäßig Behandlungen unterzogen werden, etwa einer Reinigungsbehandlung oder einem Bügeln. Der Benutzer wählt üblicherweise eine bestimmte Art der Behandlung mit entsprechenden Behandlungsparametern manuell aus. Behandlungsparameter können hierbei insbesondere die Reinigungsmittelart, Reinigungstemperatur und die Bügeltemperatur sein.

Die Identifikation der Struktur der Textilie wird üblicherweise über das optische Erscheinungsbild für das menschliche Auge vorgenommen. Die für eine Behandlung optimalen Behandlungsparameter werden oft nach der Erfahrung des Benutzers und auf Grundlage von Markierungen an der Textilie ermittelt, beispielsweise über Textilpflegezeichen auf einem an der Textilie angebrachten Etikett.

Textilien umfassen hierbei insbesondere Kleidungsstücke, Gardinen oder Bettzeug. Kleidungsstücke und Bettzeug umfassen beispielsweise Hemden, T-Shirts, Kleider, Jacken, Pullover, Hosen, Decken, Abdeckungen und Bezüge. Die Textilien können verschiedene Materialien umfassen, beispielsweise Naturfasern, Chemiefasern oder auch weitere Materialien wie Leder.

Bei der Auswahl der Behandlung der Textilie durch den Benutzer können jedoch fehlerhafte Identifikationen der Struktur der Textilie zu Fehlbehandlungen führen. Beispielsweise werden Textilien ungeeigneten Reinigungsbehandlungen unterzogen oder unter falschen Bedingungen gebügelt, insbesondere wird die Temperatur bei der Behandlung zu hoch gewählt oder ein für die Struktur der Textilie schädliches Reinigungsmittel verwendet. Hierdurch kann es zu einem erhöhten Verschleiß des Materials der Textilie oder sogar zur Zerstörung der Textilie kommen.

Die CN 204097748 U offenbart eine Textilerkennungsvorrichtung mit einem Kodeabtaster und einem Prozessor. Streifenkodes oder zweidimensionale Kodes sind an den Textilien befestigt und können über den Kodeabtaster eingelesen werden. Die Vorrichtung bestimmt hierbei aus der abgetasteten Information ein spezifisches Programm zur Reinigung der Textilie mit einer Waschmaschine. Ein entsprechendes Programm kann dann automatisch von der Waschmaschine durchgeführt werden.

Dokument WO0146509 A1 beschreibt ein Verfahren zur Bestimmung der Textilart eines Kleidungsstücks unter Verwendung einer Lichtquelle und eines optischen Detektors. Darauf aufbauend werden Reinigungsparameter zur Reinigung der Textilien angepasst. Das Dokument WO2004059075 A1 beschreibt die Verwendung eines Enzyms, um die Bildung von Pillings auf Textilien zu reduzieren.

Problematisch ist hierbei jedoch, dass ähnlich zu den handelsüblichen Textilpflegezeichen auf Etiketten jede Textilie mit einer entsprechenden Markierung bzw. einem entsprechenden Kode ausgestattet sein muss. Hierdurch wird der Produktionsaufwand der Textilien erhöht und die angebrachten Etiketten oder Markierung sind oft aus ästhetischen Gründen unerwünscht. Darüber hinaus verfügen nicht alle Textilien über entsprechende Markierungen. Etiketten können auch entfernt werden, womit die Struktur der entsprechenden Textilien wiederum nicht ohne Weiteres identifiziert werden kann. Entsprechend bleibt die Gefahr einer Fehlbehandlung bestehen.

Auch können ältere Textilien bereits Anzeichen von Verschleiß und beispielsweise sogenannte Pillings aufweisen, welche durch ein Lösen von Fasern aus dem Textilverbund entstehen und in Form von Knoten an der Textiloberfläche auftreten. Behandlungsvorgänge müssen dann ggf. entsprechend dem Materialverschleiß angepasst werden, um einen weiteren, verstärkten Materialverschleiß einzudämmen oder beispielsweise auch Pillings abzulösen um das Erscheinungsbild der Textilie wiederherzustellen. Die herkömmlichen Markierungen wie Textilpflegezeichen geben jedoch keine Hinweise bezüglich solcher wechselnder Anforderungen aufgrund von Verschleiß.

Allgemeine Beschreibung einiger beispielhafter Ausführungsformen der Erfindung Vor dem Hintergrund des dargestellten Stands der Technik ist es somit Aufgabe der Erfindung, die beschriebenen Probleme zumindest teilweise zu verringern oder zu vermeiden, das heißt die Identifikation der Struktur der Textilie zu erleichtern und entsprechend der Struktur eine optimale Behandlung der Textilie zu empfehlen.

Gemäß einem ersten Aspekt der Erfindung wird ein Verfahren entsprechend dem Anspruch 1 durchgeführt von einer oder mehreren Vorrichtungen beschrieben, das Verfahren umfassend: zerstörungsfreies Bestimmen von Strukturinformation charakteristisch für zumindest einen Teil der Struktur einer Textilie; Ermitteln von mindestens einem Behandlungsparameter der Textilie zumindest teilweise basierend auf der Strukturinformation; und Ausgeben oder Auslösen eines Ausgebens des mindestens einen Behandlungsparameters.

Gemäß einem zweiten Aspekt der Erfindung wird eine Vorrichtung entsprechend dem Anspruch 14 beschrieben, welche dazu eingerichtet ist oder entsprechende Mittel umfasst, ein Verfahren nach dem ersten Aspekt durchzuführen und/oder zu steuern. Vorrichtungen des Verfahrens gemäß dem ersten Aspekt sind oder umfassen insbesondere eine oder mehrere Vorrichtungen gemäß dem zweiten Aspekt.

Eine Strukturinformation charakteristisch für zumindest einen Teil der Struktur einer Textilie wird bestimmt. Insbesondere wird gemäß dem ersten Aspekt ein Struktursensor oder Oberflächensensor verwendet bzw. gemäß dem zweiten Aspekt ist insbesondere ein Struktursensor vorgesehen, welcher dafür eingerichtet ist, Strukturinformation über eine Textilie bereitzustellen. Der Struktursensor kann hierbei beispielsweise Information über die Form, Beschaffenheit, Erscheinung und Zusammensetzung der Textilie und des Materials der Textilie ermitteln.

Die Strukturinformation wird zerstörungsfrei bestimmt. Bei einer zerstörungsfreien Bestimmung wird das Material und die Struktur nicht oder nur unwesentlich durch die Ermittlung beeinflusst, insbesondere ändert sich das Erscheinungsbild der Textilie für den Benutzer nicht durch die Ermittlung der Strukturinformation. Entsprechend der Erfindung wird die Reaktion der Textilie auf eine Anregung, beispielsweise eine , optische Anregung untersucht, ohne dass die Anregung die Textilie irreversibel beeinflusst. Der Ermittlung der Strukturinformation und insbesondere ein Struktursensor basiert einer optischen Messung. Dies hat im Gegensatz zu invasiven bzw. zerstörungsbehafteten Verfahren, welche beispielsweise auf einer Probenentnahme der Textilie oder einer intensiven chemischen Reaktion basieren, den Vorteil, dass die Textilie bei der Durchführung des Verfahrens einem geringen Verschleiß ausgeliefert ist.

Basierend auf der Strukturinformation kann mindestens ein Behandlungsparameter für die Textilie ermittelt werden. Bei dem Behandlungsparameter handelt es sich insbesondere um eine Empfehlung für eine spezifische, auf die Struktur der Textilie abgestimmte Behandlung. Beispielsweise kann eine besonders schonende Behandlung der Textilie im Vordergrund stehen, so dass der mindestens eine Behandlungsparameter eine möglichst hohe Langlebigkeit der Textilie gewährleistet. Ebenso kann eine besonders intensive Behandlung der Textilie gewünscht sein, wobei der mindestens eine Behandlungsparameter in Hinsicht auf die Wirkung der Behandlung der spezifischen Struktur, beispielsweise der Effektivität eines Reinigungsvorgangs, optimiert ist. Auch kann eine Empfehlung über eine besonders energiesparende Behandlung von dem mindestens einen Behandlungsparameter repräsentiert werden.

Der mindestens eine Behandlungsparameter wird ausgegeben bzw. dessen Ausgabe veranlasst. Beispielsweise wird der mindestens eine Behandlungsparameter dem Benutzer auf einer Anzeigevorrichtung angezeigt, so dass der Benutzer beispielsweise eine Empfehlung über eine optimale Behandlung der Textilie bereitgestellt wird, insbesondere visuell und/oder akustisch. Der Benutzer kann dann die Behandlung durchführen.

Beispielsweise können dem Benutzer auch mehrere Sätze von Behandlungsparametern zur Verfügung gestellt werden, etwa Behandlungsparameter für besonders schonende, für besonders intensive und für besonders energiesparende Behandlungen. Dem Benutzer können auch verschiedene gleichwertige Alternativen für mögliche Behandlungsparameter zu Verfügung gestellt werden. Beispielsweise umfasst das Verfahren eine Bereitstellung einer Mehrzahl von Behandlungsparametern, wobei der Benutzer insbesondere über eine Abfrage beispielsweise von Präferenzen eine Auswahl treffen kann.

Alternativ oder zusätzlich kann mindestens ein Behandlungsparameter an eine Behandlungsvorrichtung weitergeben werden. Beispielsweise kann der mindestens eine Behandlungsparameter an eine Behandlungsvorrichtung übergeben werden, welche den entsprechenden Behandlungsparameter als Voreinstellung übernimmt und wobei der Benutzer die Behandlungsvorrichtung lediglich starten muss. Ebenso ist es denkbar, dass die Behandlungsvorrichtung mit der Ausgabe des mindestens einen Behandlungsparameters die Reinigungsbehandlung automatisch durchführt. Die Reinigungsvorrichtung kann beispielsweise über eine Dosierungsvorrichtung für Reinigungsmittel verfügen, um die Reinigungsmittelart und Reinigungsmittelmenge entsprechend der empfohlenen Behandlung automatisch bereitzustellen. Im Ergebnis wird hiermit die Benutzerfreundlichkeit des Verfahrens verbessert.

In einer Ausgestaltung des Verfahrens gemäß dem ersten Aspekt ist die Strukturinformation indikativ für Materialstruktur, Materialfarbe, Materialart, Materialverteilung, Materialverschleiß der Textilie oder eine Kombination hiervon.

Unter der Materialstruktur der Textilie wird insbesondere die Art und/oder Form eines Gewebes, einer Maschenware oder Vliesstoff bzw. Faserflor verstanden. Die entsprechende Strukturinformation kann insbesondere charakteristisch für die Art der Verflechtung von Fasern, wie diese beispielsweise über ein Weben, Wirken oder Stricken hergestellt wurde, oder charakteristisch für einen Vliesstoff sein. Ein Verflechtungsmuster bzw. ein Fadenverkreuzungsmuster und eine Fadenbindung können hierbei von der Strukturinformation umfasst werden. Fadendichte, Faserstärke, Faserlänge, Faserfeinheit und/oder Faserorientierung können insbesondere in der Strukturinformation erfasst sein. Die Materialstruktur der Textilie hat einen direkten Einfluss auf die Anforderungen an die Behandlung der Textilie, beispielsweise kann ein Vliessstoff andere Anforderungen an eine Reinigungsbehandlung stellen als eine gewirkte oder gewebte Struktur.

Die Materialfarbe kann indikativ für eine oder mehrere Farben der Textilie sein. Beispielsweise gibt die Strukturinformation eine mittlere Färbung, eine Farbverteilung und/oder ein Maß für die Homogenität der Farbverteilung an. Beispielsweise enthält die Strukturinformation Werte in einem Farbraum wie einem RGB-Farbraum und/oder einem L*a*b*-Farbraum. Insbesondere wird für die Ermittlung der Strukturinformation indikativ für die Farbe der Textilie eine Farbreferenz genutzt, beispielsweise eine Farbkarte, welche mit der Strukturinformation aufgenommen wird. Die Ermittlung der Strukturinformation indikativ für die Farbe der Textilie kann einen Weißabgleich umfassen, beispielsweise anhand einer Referenz wie einer Graukarte, welche mit der Strukturinformation aufgenommen wird. Die Ermittlung der Strukturinformation indikativ für die Farbe der Textilie kann insbesondere Aspekte des in der WO2016/126470 A1 beschriebenen Verfahrens umfassen.

Insbesondere enthält die Strukturinformation ein Maß für den Glanz der Struktur der Textilie, welche beispielsweise über die Reflektion der Oberfläche der Textilie, insbesondere über die diffuse Reflektion der Oberfläche charakterisiert wird. Beispielsweise umfasst die Strukturinformation eine Winkelabhängigkeit der Reflektivität der Oberfläche der Textilie. Insbesondere umfasst die Strukturinformation ein Maß für den Glanz der Struktur der Textilie an mehreren Stellen der Oberfläche, insbesondere an sauberen und verunreinigten Stellen.

Unter der Materialart wird insbesondere die Zusammensetzung zumindest eines Teils des Materials der Textilie verstanden. Beispielsweise ist die Strukturinformation indikativ für Naturfasern, Chemiefasern oder natürliche Materialien wie Wolle oder Leder in der Textilie. Die Materialart hat ebenso erheblichen Einfluss auf eine optimale Behandlung der Textilie, beispielsweise eine Reinigungsbehandlung oder ein Bügeln.

Mit der Materialverteilung der Textilie kann beispielsweise erfasst werden, ob die Textilie ein Mischgewebe aus unterschiedlichen Faserarten oder Fasermaterialien aufweist und/oder ob Teilbereiche der Textilie aus einem anderen Material gefertigt sind. Hierbei kann das Verhältnis der verschiedenen Materialien zueinander, beispielsweise ein Dichteverhältnis, Massenverhältnis oder Flächenverhältnis erfasst werden. Weiter in der Strukturinformation enthalten sein können die Art und Anzahl von Verbindungsstellen, beispielsweise Nähte, Verschweißungen oder Klebestellen.

Mit der Strukturinformation indikativ für den Materialverschleiß kann insbesondere festgehalten werden, ob sich Pillings, Risse, Löcher, Abnutzungen oder sonstige Strukturschäden an der Textilie befinden. Insbesondere für Pillings, welche durch ein Lösen von Fasern aus dem Textilverbund entstehen und in Form von Knoten an der Textiloberfläche auftreten, kann die Art, Form, Größe bzw. Höhe, Anzahl und/oder Verteilung des Materialverschleißes erfasst werden. Durch die Ermittlung mindestens eines Behandlungsparameters basierend auf dem Materialverschleiß kann die Behandlung der Textilie entsprechend dem Materialverschleiß angepasst werden, um einen weiteren, verstärkten Materialverschleiß einzudämmen oder beispielsweise auch Pillings abzulösen um das Erscheinungsbild der Textilie wiederherzustellen.

In einer weiteren Ausgestaltung des Verfahrens gemäß dem ersten Aspekt ist die Strukturinformation indikativ für das Vorhandensein und/oder Art von Verschlussmitteln, von Beschichtungsmaterial und/oder von Applikationen in, an und/oder auf der Textilie.

Unter Verschlussmitteln auf der Textilie werden insbesondere Reißverschlüsse, Klettverschlüsse, Knöpfe oder ähnliche Anordnungen verstanden, welche insbesondere dafür eingerichtet sind, eine Verbindung von Teilen der Textilie über einen Formschluss herzustellen und welche lösbar ausgestaltet sein können.

Die Textilie kann ein oder mehrere Beschichtungsmaterialien aufweisen, insbesondere sind die Fasern beschichtet oder eine Beschichtung ist auf der Struktur des Materials der Textilie, beispielsweise auf dem Gewebe aufgebracht. Die Beschichtung kann beispielsweise eine funktionelle Schicht wie eine Schutzschicht oder Abdichtschicht sein oder Aussehen oder Haptik der Textilie verändern.

Textilien, insbesondere Kleidungsstücke, können weiter Applikationen wie Aufdrucke, Pailletten, Spitze, Flicken oder ähnliches aufweisen, was ebenso mit der Strukturinformation charakterisiert werden kann. Ebenso können Funktionstextilien funktionelle Elemente als Applikationen aufweisen oder elektronische Elemente in der Textilie oder an der Oberfläche der Textilie angeordnet sein.

Ist die Strukturinformation indikativ für solche Verschlussmittel, Beschichtungen und/oder Applikationen auf der Textilie, kann bei einer Behandlung auch auf die Schonung der entsprechenden Elemente Rücksicht genommen werden. Einerseits kann ein Verschleiß solcher Verschlussmittel, Beschichtungen und/oder Applikationen bei einer Behandlung vermindert und anderseits auch beispielsweise eine Ablösung von Beschichtungen oder Applikationen vermieden werden.

In einer weiteren Ausgestaltung des Verfahrens gemäß dem ersten Aspekt ist der mindestens eine Behandlungsparameter indikativ für eine Empfehlung einer Vorbehandlung, einer Reinigungsbehandlung und/oder einer Endbehandlung der Textilie.

Eine Vorbehandlung kann hierbei eine Vorreinigung, eine Applikation von Vorbehandlungsmitteln oder eine bestimmte Anordnung der Textilie umfassen. Beispielsweise gibt mindestens ein Behandlungsparameter eine Vorreinigung oder Vorwäsche an, insbesondere ein Einweichen der Textilie in einer bestimmten Lösung oder ein Vorreinigungsprogramm einer Reinigungsvorrichtung. Verschiedene Vorbehandlungsmittel können für eine manuelle oder automatische Applikation vorgesehen sein, beispielsweise wird das Aufbringen eines Fleckenlösers oder eines Bleichmittels angegeben. Weiter kann eine Anordnung der Textilie insbesondere darin gegeben sein, dass die Textilie vor der eigentlichen Behandlung "auf links" gedreht oder in einer weiteren Vorrichtung, etwa in einem Wäschebeutel angeordnet werden sollte. Weiter kann die Vorbehandlung auch ein Schließen der Verschlussmittel umfassen, beispielsweise kann der Benutzer einen Hinweis auf das Schließen eines Reißverschlusses für eine nachfolgende Behandlung erhalten.

Als Behandlung der Textilie kann beispielsweise vorgesehen sein, dass die Textilie gefärbt wird oder einer Schonungsbehandlung unterzogen wird. Beispielsweise kann auf Grundlage der Strukturinformation eine Färbungsempfehlung ermittelt werden, wobei die Textilie gemäß dem mindestens einen Behandlungsparameter durch eine Färbung eine Auffrischung oder Änderung der Farbgebung erhält.

In einer vorteilhaften Ausgestaltung des Verfahrens gemäß dem ersten Aspekt umfasst die Behandlung eine Reinigungsbehandlung, insbesondere eine Waschbehandlung durchgeführt auf einer Reinigungsvorrichtung, beispielsweise einer Waschmaschine.

Die Waschmaschine kann in verschiedenen Ausgestaltungsformen vorliegen. Man unterscheidet Toplader, bei denen die Ladeluke an der Oberseite liegt, und Frontlader, bei denen ein Bullauge als Ladeluke an der Vorderseite dient. Vorteil des Topladers ist, dass die Abdichtung der Tür einfacher ausgeführt und die Trommel auf zwei Seiten durch Wälzlager abgestützt sein kann, ein Toplader lässt sich auch in sehr engen Räumen aufstellen, wo nicht genügend Platz zum Öffnen einer vorderen Tür zur Verfügung steht. Ein Frontlader hingegen bietet auf der Oberseite Platz für z. B. einen Wäschetrockner oder für eine Arbeitsfläche und wird deswegen gelegentlich anstelle eines Unterschranks in eine Küchenzeile eingebaut.

Die amerikanischen Toplader haben immer eine rotierende Trommel und Mischelemente (Agitator oder Discs), wobei sich die Mischelemente mit oder gegen die Trommeldrehrichtung bewegen können. Die Maschinen können eine Laugenumwälzung und Sprühvorrichtungen für die Lauge aufweisen. Grundsätzlich werden Deep Fill und HE-Toploader unterschieden. Deep Fill Toplader arbeiten mit vorgegebenen Wasserniveaus, haben also keine Beladungserkennung. HE Maschinen verfügen in der Regel über eine Beladungserkennung und steuern danach die Wassermengen. In der Regel haben die Maschinen keine eingebaute Heizung, sondern werden an Warm- und Kaltwasser angeschlossen.

In einer Ausgestaltung ist der mindestens eine Behandlungsparameter indikativ für eine Reinigungsmittelart, eine Reinigungsmittelmenge, eine Reinigungstemperatur, einen Reinigungsvorrichtungstyp, Einstellungen einer Reinigungsvorrichtung oder eine Kombination hiervon.

Reinigungsmittel werden beispielsweise im Haushalt für die Reinigung unterschiedlicher Objekte eingesetzt. Beispielsweise wird für Waschmaschinen ein Reinigungsmittel, zum Beispiel ein Waschmittel, für die Reinigung von Textilien eingesetzt. Unter einem Reinigungsmittel sollen jedoch ebenfalls auch Reinigungshilfsmittel oder Reinigungszusatzmittel, wie beispielsweise ein Bleichzusatzmittel, ein Weichspüler oder Wäschestärke, verstanden werden. Ein Reinigungsmittel kann zudem eine Flüssigkeit, ein disperses System, zum Beispiel ein Gel oder Schaum, oder ein Feststoff, insbesondere ein Tab, Pulver oder Granulat, sein. Die genannten Reinigungsmittel können ebenso für die Vorbehandlung oder Endbehandlung der Textilie herangezogen werden.

Ein Reinigungsmittel kann beispielsweise eine oder mehrere Komponenten aus der Gruppe von Komponenten umfassend Tenside, Alkalien, Builder, Vergrauungsinhibatoren, optische Aufheller, Enzyme, Bleichmittel, Soil-Release-Polymere, Füller, Weichmacher, Duftstoffe, Farbstoffe, Pflegestoffe, Säuren, Stärke, Isomalt, Zucker, Zellulose, Zellulosederivate, Carboxymethylcellulose, Polyetherimid, Silikonderivate und/oder Polymethylimine aufweisen.

Ein Reinigungsmittel kann ferner einen oder mehrere weitere Bestandteile umfassen. Diese Bestandteile schließen ein, sind aber nicht beschränkt auf die Gruppe bestehend aus Bleichaktivatoren, Komplexbildnern, Gerüststoffen, Elektrolyten, nichtwässrigen Lösungsmitteln, pH-Stellmitteln, Parfümträgern, Fluoreszenzmitteln, Hydrotropen, Silikonölen, Bentoniten, Antiredepositionsmitteln, Einlaufverhinderern, Knitterschutzmitteln, Farbübertragungsinhibitoren, , antimikrobiellen Wirkstoffen, Germiziden, Fungiziden, Antioxidantien, Konservierungsmitteln, Korrosionsinhibitoren, Antistatika, Bittermitteln, Bügelhilfsmitteln, Phobier- oder Imprägniermitteln, Quell- oder Schiebefestmitteln und/oder UV-Absorbern.

Der mindestens eine Behandlungsparameter kann die Reinigungsmittelart repräsentieren und somit indikativ für die Zusammensetzung des empfohlenen Reinigungsmittels sein. Wenn beispielsweise ein gewisser Anteil Wolle in der Struktur der Textilie enthalten oder Applikationen auf der Textilie vorhanden sind, kann dem Benutzer die Verwendung von entsprechend schonenden Reinigungsmitteln empfohlen werden.

Der mindestens eine Behandlungsparameter kann die Reinigungsmittelmenge repräsentieren und insbesondere eine absolute Menge des Reinigungsmittels angeben. Ebenso kann eine relative Menge des Reinigungsmittels mittels des mindestens einen Behandlungsparameters angezeigt werden, beispielsweise bezogen auf die Masse der zu reinigenden Textilien bzw. ein Flottenverhältnis oder eine Reinigungsmittelmenge bezogen auf ein zur Reinigung einzusetzendes Wasservolumen.

Mit einem für die Reinigungstemperatur repräsentativen Behandlungsparameter kann eine für die bestimmte Struktur der Textilie optimale Temperatur zur Reinigung angeben werden, insbesondere in Kombination mit einer Reinigungsmittelart. Die Reinigungstemperatur kann hierbei einerseits hoch genug sein, um eine möglichst vollständige Reinigung der Textilie zu gewährleisten und anderseits im Hinblick auf den Energieaufwand und einer Schonung der Textilie niedrig gehalten werden.

Unter einer Reinigungsvorrichtung wird insbesondere eine Waschmaschine, insbesondere automatische Haushaltswaschmaschine verstanden. Hierbei kann ein Behandlungsparameter einen bestimmten Typ einer solchen Reinigungsvorrichtung angeben. Denkbar ist auch, dass der Behandlungsparameter zumindest teilweise manuell durchzuführende Reinigungsbehandlungen vorgibt, etwa eine Handwäsche. Auch kann der mindestens eine Behandlungsparameter Einstellungen einer Reinigungsvorrichtung umfassen, beispielsweise ein Programm einer automatischen Haushaltswaschmaschine oder eine Sequenz solcher Programme. Die Dauer der Behandlung kann ebenfalls über den Behandlungsparameter wiedergegeben sein, um eine besonders schonende Reinigung der Textilie zu erreichen.

Die Empfehlung der Behandlungsparameter einer Endbehandlung kann beispielsweise eine Trocknung oder Glättung, insbesondere ein Bügeln der Textilie betreffen. Der mindestens eine Behandlungsparameter kann hierbei unter anderem die Behandlungstemperatur, Behandlungslänge und/oder einen Behandlungsmodus angeben. Eine Endbehandlung erfolgt beispielsweise nach der Reinigungsbehandlung. Ebenso ist es möglich, Endbehandlungen wie Trocknung oder Glättung unabhängig von einer Reinigungsbehandlung durchzuführen.

Im Ergebnis kann mit dem mindestens einen Behandlungsparameter indikativ für eine Empfehlung einer Vorbehandlung, einer Reinigungsbehandlung und/oder einer Endbehandlung der Textilie dem Benutzer die Identifikation der Struktur der Textile und deren Behandlung erheblich erleichtert werden. Insbesondere bei Textilien, welche über keine Markierungen für deren Behandlung verfügen, oder bei verschlissenen Textilien kann über das Verfahren eine im Hinblick auf die Schonung des Materials der Textilie optimale Behandlung empfohlen werden.

In einer weiteren Ausgestaltung umfasst das Verfahren weiterhin: Durchführen oder Veranlassen der Durchführung einer Behandlung der Textilie entsprechend dem mindestens einen ermittelten Behandlungsparameter über mindestens eine Behandlungsvorrichtung, insbesondere eine Reinig ungsvorrichtu ng.

In einer weiteren Ausgestaltung des Verfahrens gemäß dem ersten Aspekt wird die Strukturinformation vor, während und/oder nach einer Behandlung der Textilie bestimmt. Mit einer Bestimmung vor der Behandlung kann beispielsweise dem Benutzer vor einer durchzuführenden Reinigungsbehandlung eine Empfehlung über die zu verwendenden Behandlungsparameter gegeben werden.

Bei einer Bestimmung der Strukturinformation während der Behandlung kann die Behandlung beispielsweise dynamisch durchgeführt werden, d.h. eine Behandlungsvorrichtung kann sich während der Behandlung an die gerade zu behandelnde Struktur eines Teilabschnitts der Textilie anpassen, insbesondere indem die Behandlungsparameter kontinuierlich ermittelt werden. Beispielsweise passt eine Bügelvorrichtung während der Bewegung über die Oberfläche der Textilie die Behandlungsparameter, beispielsweise der Temperatur, an die Struktur des momentan zu bügelnden Oberflächenabschnitts der Textilie an.

Mit einer Bestimmung der Strukturinformation nach einer Behandlung kann beispielsweise das Ergebnis bzw. die Effektivität einer Behandlung festgehalten und überprüft werden.

Entsprechend der Erfindung wird zur Bestimmung der Strukturinformation mindestens ein optisches Sensorelement verwendet. Entsprechend weist die Vorrichtung einen Struktursensor bzw. Oberflächensensor aufweisen, welcher mindestens ein optisches Sensorelement ist.

Mit einem optischen Sensorelement kann mit der Bildinformation das Erscheinungsbild der Textilie und dessen Struktur ermittelt werden, beispielsweise über Werte einer räumlich aufgelösten Intensitätsverteilung mit oder ohne Farbauflösung. Das optische Sensorelement kann hierbei die Identifikation der Struktur ermöglichen und insbesondere eine Bildauflösung bereitstellen, welche die des menschlichen Auges übersteigt, beispielsweise in der Energieauflösung, der räumlichen Auflösung und/oder im Hinblick auf den berücksichtigten Wellenlängenbereich.

Unter einem optischen Sensor werden hierbei Sensoren verstanden, welche eine Intensität einfallender Strahlung, insbesondere elektromagnetischer Strahlung im sichtbaren Bereich und ggf. darüber hinaus, ermitteln können. Insbesondere ist der optische Sensor dafür eingerichtet, eine räumliche Auflösung und/oder eine Farbinformation der in den Sensor einfallenden Strahlung bereitzustellen. Der optische Sensor kann einen Bildsensor umfassen, insbesondere einen digitalen Bildsensor. Zur Ermittlung der einfallenden Strahlung kann insbesondere mindestens ein Halbleiterelement, Dioden, CCD-Elemente, beispielsweise ein Bayer-Sensor, oder CMOS-Elemente, beispielsweise ein Sensor des Typs Foveon X3, verwendet werden. Der optische Sensor kann optische Filter und insbesondere ein Spektrometer enthalten. Es können weitere optische Elemente wie Linsen und/oder Filter, beispielsweise ein externer Monochromator vorgesehen sein.

Gemäß einer Ausgestaltung ist die Strukturinformation repräsentativ für spektrale Anteile eines Spektralbilds. Die spektralen Anteile können innerhalb des sichtbaren Bereichs liegen. Ist gemäß einer weiteren Ausgestaltung des Verfahrens die Strukturinformation repräsentativ für spektrale Anteile eines Spektralbilds, wobei mindestens einer der spektralen Anteile außerhalb des sichtbaren Energiebereichs liegt, kann die Struktur der Textilie mit erhöhter Genauigkeit in die Ermittlung des Behandlungsparameters einfließen. Dadurch, dass nicht sichtbare spektrale Anteile berücksichtigt werden, können auch verschiedene Strukturen von Textilien identifiziert werden, obwohl diese für das Auge nicht zu unterscheiden sind. Zur Ermittlung von spektralen Anteilen des Spektralbilds kann insbesondere ein Abgleich mit einem Empfindlichkeitsspektrums des Struktursensors erfolgen, beispielsweise anhand von vorgegebenen Einstellungen oder durch Abgleich mit einer Referenz.

Insbesondere ist die Strukturinformation repräsentativ für spektrale Anteile eines Spektralbilds im ultravioletten Energiebereich. Ebenfalls können spektrale Anteile im infraroten Energiebereich berücksichtigt werden. Die Strukturinformation ist insbesondere repräsentativ für spektrale Anteile eines Spektralbilds vom infraroten Energiebereich bis zum ultravioletten Energiebereich, beispielsweise zumindest für spektrale Anteile eines Spektralbilds mit Wellenlängen von 1400 nm bis 315 nm, bevorzugt für Wellenlängen von 3000 nm bis 280 nm, weiter bevorzugt für Wellenlängen von 5000 nm bis 200 nm. Die Strukturinformation ist insbesondere repräsentativ für spektrale Anteile eines Spektralbilds im (nahen) infraroten Energiebereich, beispielsweise für Wellenlängen von 700 nm bis 2400 nm, insbesondere 780 nm bis 2000 nm, insbesondere bis 1450 nm.

Denkbar ist ebenfalls die Verwendung von monochromen Sensoren ohne Farbauflösung. Ebenso können Sensoren verwendet werden, welche auf bestimmte Wellenlängenbereiche beschränkt sind, beispielsweise basierend auf mindestens einer Photodiode und/oder mindestens einem LED-Element.

In einer Ausgestaltung des Verfahrens umfasst das Verfahren weiterhin: Durchführen einer Referenzierung der Ermittlung der Strukturinformation. Beispielsweise kann zur Ermittlung von spektralen Anteilen des Spektralbilds insbesondere ein Abgleich mit einem Empfindlichkeitsspektrum erfolgen, beispielsweise anhand von vorgegebenen Einstellungen oder durch Abgleich mit einer Referenz. Die Referenz kann insbesondere als Karte ausgestaltet sein, beispielsweise in Form einer Farbkarte, Graukarte und/oder eines Größenmaßstabs, welche auf die Textilie gelegt werden können. Ebenso kann eine Referenz in und/oder an einer Behandlungsvorrichtung angebracht sein. Beispielsweise ist eine Oberfläche im Inneren der Behandlungsvorrichtung, etwa die Oberfläche eines Reinigungsbehälters, mit einer Referenz versehen. Die Referenz kann auch Teil einer Umverpackung eines Mittels zur Durchführung eines Wasch-, Reinigungs-, Pflege- oder Färbevorgangs sein z.B. in Form eines Aufdrucks, eines ablösbaren Teils der Verpackung oder als digitale Information z.B. in Form eines elektronischen Labels.

Entsprechend der Erfindung stellt das optische Sensorelement eine dreidimensionale räumliche Auflösung bereit. Mit einer dreidimensionalen räumlichen Auflösung kann die Genauigkeit der Strukturbestimmung der Textilie weiter erhöht werden. Denkbar ist die Verwendung von mehreren Aufnahmen aus verschiedenen Perspektiven über den gleichen optischen Sensor bzw. die gleiche Sensoranordnung. Ebenso können speziell für eine dreidimensionale Auflösung ausgebildete optische Elemente wie Aufsatzlinsen oder Objektive vorgesehen sein oder eine 3D-Kamera verwendet werden. Zusätzliche optische Elemente, beispielsweise Aufsatzlinsen oder Objektive, können auch auf herkömmlichen, im Wesentlichen zweidimensionalen optischen Sensoren, beispielsweise digitale Kameras oder in mobilen Geräten integrierten Kameras angeordnet werden. Damit können auch bereits vorhandene Vorrichtungen für eine dreidimensionale Auflösung nachgerüstet werden (Retrofitting). Mit der dreidimensionalen Auflösung kann die Textilstruktur, beispielsweise die Form und Anordnung des Gewebes, der Maschen oder des Vliesstoffs, eingehender bestimmt werden und somit eine umfassendere und genauere Strukturinformation erhalten werden.

Insbesondere umfasst die Strukturinformation einen oder mehrere Parameter, eine Größeninformation, beispielsweise einer räumlichen Ausdehnung bzw. des Volumens der Textilie. Insbesondere kann auch die Dimensionierung der Struktur der Textilie, beispielsweise die Faserstärke, Faserdichte, Maschengröße, Maschendichte, die Größe von Abnutzungen wie beispielsweise Pillings bestimmt werden. Insbesondere wird die Dimensionierung in Verbindung mit einer Referenz bestimmt.

Insbesondere umfasst die Strukturinformation ein Maß für die Rauigkeit der Oberfläche, beispielsweise in Verbindung mit einer dreidimensionalen räumlichen Auflösung. Das Maß für die Rauigkeit kann beispielsweise die mittlere Rauheit R_{A}, die mittlere Rautiefe Rz und/oder die maximale Rautiefe Rₘₐₓ umfassen. Über die Rauigkeit kann die Struktur der Textilie weiter bestimmt werden. Insbesondere kann die Rauigkeit auch ein Hinweis auf den Verschleißgrad der Textilie sein.

Zur Ermittlung der Strukturinformation über einen Struktursensor, insbesondere umfassend ein optisches Sensorelement, können auch eine oder mehrere Referenzen verwendet werden. Die Referenzen können vor, nach und/oder gleichzeitig mit der Strukturinformation über den Struktursensor erfasst werden. Beispielsweise wird eine Kalibrierungskarte verwendet, welche Farbreferenzen und/oder Größenreferenzen umfasst. Über eine Erfassung der Kalibrierungskarte kann somit beispielsweise die Strukturinformation hinsichtlich der Farbe und der Abmessungen, insbesondere bei einer dreidimensionalen räumlichen Auflösung, genauer bestimmt werden.

In einer besonders einfachen Ausgestaltung umfasst das mindestens eine optische Sensorelement mindestens ein kameraartiges Element und stellt eine Bildinformation bereit. Entsprechend können digitale Kameras oder in mobilen Geräten integrierte Kameras für das Verfahren herangezogen werden bzw. als mindestens eine Vorrichtung zur Durchführung des Verfahrens dienen. Dabei können Aufsätze für eine dreidimensionale räumliche Auflösung an dem kameraartigen Element verwendet werden.

In einer Ausgestaltung umfasst die Bildinformation mindestens zwei Einzelbilder der Textilie. Die Einzelbilder können hierbei eine zeitliche Abfolge darstellen, beispielsweise eine oder mehrere Filmsequenzen, oder auch eine Variation der Position und Perspektive des kameraartigen Elements wiedergeben. Hierdurch kann die Genauigkeit der Strukturinformation weiter erhöht werden. Insbesondere kann, wie oben bereits beschrieben, über eine Mehrzahl von Einzelbildern eine dreidimensionale räumliche Auflösung erreicht werden. Ebenso kann die Struktur der Textilie von verschiedenen Seiten erfasst werden, beispielsweise von Vorderseite, Rückseite, Außenseite und/oder Innenseite. Insbesondere können auch Verunreinigungen auf der Textilie von verschiedenen Seiten erfasst werden.

In einer weiteren Ausgestaltung des Verfahrens gemäß dem ersten Aspekt umfasst die Strukturinformation der Textilie eine Bildinformation einer Beschriftung einer Markierung der Textilie. Unter einer Markierung wird insbesondere ein Etikett verstanden, welches insbesondere an der Textilie angebracht ist. Die Markierung kann auch teilweise oder ganz auf der Struktur der Textilie selbst angeordnet sein. Die Markierung ist insbesondere indikativ für Parameter charakteristisch für die Struktur der Textilie, insbesondere die Materialstruktur, Materialart, Materialverteilung, Farbe der Textilie, Materialverschleiß der Textilie, Art und/oder Form eines Gewebes, einer Maschenware, Vliesstoff bzw. Faserflor oder eine Kombination hiervon. Die Markierung ist insbesondere indikativ für Parameter charakteristisch für einen Behandlungsparameter.

Die Markierung umfasst hierbei eine Beschriftung. Die Beschriftung umfasst insbesondere Textzeichen und/oder Pflegesymbole. Pflegesymbole können hierbei standardisierten Pflegesymbolen entsprechend, beispielsweise Pflegesymbolen nach dem Ginetex-Standard. Insbesondere werden von einer Beschriftung hierbei Kodes, beispielsweise zweidimensionale oder dreidimensionale Strichkodes ausgenommen. Somit kann die Strukturinformation anhand eines Kennzeichens, welches insbesondere für die Zusammensetzung der Struktur der Textilie charakteristisch ist, beispielsweise ein Kennzeichen nach dem Textilkennzeichengesetz (TKG), und/oder einem Pflegehinweisetikett, welches bevorzugte Behandlungen der Textilie vorgibt, bereitgestellt werden.

Insbesondere umfasst das Verfahren gemäß dem ersten Aspekt eine Texterkennung und/oder Klassifizierung der Beschriftung. Hierzu können eine oder mehrere Aktionen einer Texterkennung durchgeführt werden, beispielsweise in einem *optical character recognition* (OCR)-Verfahren und/oder einem *intelligent character recognition* (ICR)-Verfahren, um die Beschriftung der Markierung aufzulösen. Insbesondere wird die Strukturinformation über ein oder mehrere Filter bearbeitet, beispielsweise zur Einstellung von Helligkeit, Kontrast und/oder Farbtiefe. Die Strukturinformation kann einer Klassifizierung zur Texterkennung und/oder zur Identifikation von Pflegesymbolen unterzogen werden, insbesondere in Kombination mit einer Kontextanalyse. Dies ist insbesondere bei Markierungen an Textilien hilfreich, da die Markierungen bereits durch das Alter der Textilien beeinflusst sein können und beispielsweise bereits verblasst oder unvollständig sind. Mit Hilfe der Kontextanalyse kann somit eine unvollständige Beschriftung ergänzt werden. Mit einer Texterkennung können beispielsweise übliche Beschriftungen auf Etiketten an Textilien erkannt werden, so dass die Textilien über keine speziellen Etiketten verfügen müssen.

Insbesondere umfasst die Ermittlung des mindestens einen Behandlungsparameters eine Korrelation der Strukturinformation. Insbesondere können Anteile der Strukturinformation miteinander korreliert werden, Hierbei können Anteile repräsentativ für eine Bildinformation der Struktur der Textilie, repräsentativ für spektrale Anteile, insbesondere umfassend den (nahen) Infrarotbereich, repräsentativ für Bildinformationen einer Beschriftung auf einer Markierungen oder Kombinationen hiervon miteinander korreliert werden. Beispielsweise wird über die Bildinformation der Struktur die Art des Gewebes, über die spektralen Anteile die Zusammensetzung des Materials der Struktur ermittelt und die entsprechenden Parameter mit der Auswertung der Bildinformation der Markierung abgeglichen.

In einer weiteren Ausgestaltung des Verfahrens ist die Strukturinformation repräsentativ für ein hyperspektrales Bild. Unter einer für ein hyperspektrales Bild repräsentativen Strukturinformation wird insbesondere verstanden, dass die Strukturinformation als Intensitätsverteilung Intensitätswerte in mehreren Kanälen für verschiedene Energieintervalle aufweist, wobei mindestens zwei der Energieintervalle aneinander anschließen oder überlappen. Insbesondere kann ein hyperspektrales Bild von einem multispektralen Bild darin abgegrenzt werden, dass ein multispektrales Bild zwar ebenfalls Intensitätswerte in mehreren Kanälen für verschiedene Energieintervalle aufweist, wobei jedoch die Energieintervalle voneinander beabstandet liegen, d.h. bei einem multispektralen Bild werden Intensitäten von einzelnen, voneinander abgegrenzten Energien wiedergegeben. Dagegen werden bei einem hyperspektralen Bild insbesondere "benachbarte" Intensitätswerte wiedergegeben, indem mindestens zwei der Energieintervalle aneinander anschließen oder überlappen. Ein hyperspektrales Bild kann somit zumindest teilweise ein kontinuierliches Spektrum wiedergeben. Eine Strukturinformation repräsentativ für ein hyperspektrales Bild hat insbesondere den Vorteil, dass auch für das Auge nicht sichtbare Informationen, welche indikativ für die Zusammensetzung der Verunreinigung und/oder die Struktur der Textilie sind, erfasst werden können.

Die Strukturinformation kann hierbei Werte in mindestens 20 Kanälen umfassen, wobei jeder Kanal eine Intensität für ein Energieintervall repräsentiert. Werden Werte der Strukturinformation in mindestens 20 Kanälen bereitgestellt, kann die Auflösung des Spektralbilds und damit auch die Genauigkeit der Ermittlung des mindestens einen Behandlungsparameters verbessert werden. Insbesondere umfasst die Strukturinformation mindestens 20 Kanäle bis 250 Kanäle, womit eine genauere Abhängigkeit des Behandlungsparameters von der Zusammensetzung der Verunreinigung und/oder der Struktur der Textilie erreicht wird. Mit mindestens 20 Kanälen kann in einer für ein Spektralbild, insbesondere für ein hyperspektrales Bild repräsentativen Strukturinformation erreicht werden, dass Energieintervalle repräsentiert werden, welche mit dem menschlichen Auge, welches lediglich über drei Kanäle im sichtbaren Bereich verfügt, nicht aufgelöst werden können.

In einer weiteren Ausgestaltung des Verfahrens gemäß dem ersten Aspekt wird zur Bestimmung der Strukturinformation der Textilie mindestens ein akustisches Sensorelement verwendet. Die Vorrichtung gemäß dem zweiten Aspekt kann entsprechend einen Struktursensor umfassend mindestens ein akustisches Sensorelement aufweisen.

Ein akustisches Sensorelement kann hierbei den bei einer Anregung der Textilie ausgehenden Schall erfassen und zur Bestimmung einer Strukturinformation heranziehen, womit eine zerstörungsfreie Strukturbestimmung möglich wird. Insbesondere werden mit dem akustischen Sensorelement Schallwellen mit verschiedenen Frequenzen oder Frequenzbereichen detektiert und in einer Auswertung der Strukturinformation zur Ermittlung des mindestens einen Behandlungsparameters herangezogen. Vorteilhafterweise umfassen diese Frequenzen bzw. Frequenzbereiche auch Frequenzen außerhalb des hörbaren Bereichs von 16 Hz bis 20 kHz und liegen beispielsweise im Ultraschallbereich, so dass der Benutzer von den ausgehenden Schallwellen nicht beeinträchtigt wird. Das akustische Sensorelement kann insbesondere Schall an der Textilie in Transmission und/oder Reflexion messen.

Aus einer Analyse des ermittelten Schalls, insbesondere aus einer frequenzabhängigen Analyse, kann die Struktur der Textilie charakteristisch identifiziert werden. Der Analyse können insbesondere Werte für die folgenden physikalischen Größen einzeln oder in Kombination zugrunde liegen:
- Schallschnelle (bzw. Geschwindigkeit) v;
- Schallfluss (bzw. über eine Fläche integrierte Schallschnelle) q;
- Schalldruck *p*;
- Schallimpedanz (bzw. Schallwiderstand) Z;
- Schallgeschwindigkeit c, insbesondere richtungsabhängige Schallgeschwindigkeit c in der Struktur der Textilie; und
- Auslenkung/Amplitude ξ des Schalls.

Gemäß einer Ausgestaltung des Verfahrens gemäß dem ersten Aspekt umfasst das Verfahren weiterhin ein Unterziehen der ermittelten Strukturinformation einem Bearbeitungsalgorithmus.

Hierdurch kann zum einen eine bessere Unterscheidung von verschiedenen Strukturen der Textilie erreicht werden. Beispielsweise wird die ermittelte Strukturinformation einem Konvertierungsalgorithmus unterzogen. Beispielsweise kann eine Konvertierung der ermittelten Strukturinformation (beispielsweise einer oder mehrerer Bildinformationen) von einem ersten Darstellungsraum in einen zweiten Darstellungsraum erfolgen, beispielsweise von einem ersten Farbraum zu einem zweiten Farbraum. Beispiele für Farbräume sind etwa ein RGB-Farbraum oder ein L*a*b*-Farbraum. Beispielsweise erfolgt eine Konvertierung der ermittelten Bildinformation von einem RGB-Farbraum in einen L*a*b*-Farbraum.

Unter einem RGB-Farbraum ist ein additiver Farbraum, der Farbwahrnehmungen durch das additive Mischen dreier Grundfarben (Rot, Grün und Blau) nachbildet, zu verstehen. Ein Beispiel für einen L*a*b*-Farbraum ist beispielsweise der CIELAB-Farbraum, der in der EN ISO 11664-4 "Colorimetry -- Part 4: CIE 1976 L*a*b* Colour space" (CIE 1976 Farbraum) genormt ist. Vorteilhaft ist hier, dass Farben unabhängig von der Art ihrer Erzeugung oder Wiedergabetechnik so definiert werden, wie sie von einem Normalbeobachter bei einer Standard-Lichtbedingung wahrgenommen werden (Geräteunabhängigkeit und Wahrnehmungsbezogenheit).

Zur Ermittlung der Behandlungsparameter bzw. zur Analyse der Bildinformation kann insbesondere eine Auswertung von Farbdifferenzen zwischen Pixeln einer Bildinformation vorgenommen werden.

Hierzu können insbesondere auf der Farbdifferenz bzw. dem Farbabstand Δ*E* basierende Methoden herangezogen werden. Insbesondere wird die Berechnung von Δ*E* im CIELAB-Farbraum vorgenommen. Gleichfalls können zur Ermittlung des mindestens einen Behandlungsparameters die Helligkeit in der Bildinformation herangezogen werden.

Insbesondere für eine frequenzabhängige Analyse der Strukturinformation kann eine Bearbeitung umfassend eine Fourier-Transform-Analyse oder ähnliche mathematische Methoden vorgenommen werden. Die Analyse kann hierbei für bestimmte Frequenzen oder Frequenzbereiche verwendet werden. Ebenso kann die zeitliche Abhängigkeit der Strukturinformation in Betracht gezogen werden. Insbesondere bei einer frequenzabhängigen Analyse kann beispielsweise eine Bearbeitung eine Kurzzeit-Fourier-Transform-Analyse umfassen.

In einer Ausgestaltung des Verfahrens umfasst die Ermittlung des mindestens einen Behandlungsparameters einen Vergleich der Strukturinformation mit Vergleichswerten. Entsprechende Vergleichswerte können in einer Datenbank hinterlegt sein. Die Strukturinformation kann einer Klassifizierung unterzogen werden, wobei der mindestens eine Behandlungsparameter durch ein Ergebnis der Klassifizierung erhalten oder beeinflusst wird. Eine Klassifizierung kann beispielsweise auf einem Vergleich der Strukturinformation mit einer Datenbank von bereits bekannten Strukturinformationen beruhen. Weiter können den entsprechenden Vergleichswerten bestimmte Behandlungsparameter zugeordnet sein.

Die Ermittlung des Behandlungsparameters kann einen oder mehrere Schritte einer Merkmalsextraktion und/oder eines Merkmalsabgleichs umfassen. Beispielsweise werden Methoden herangezogen, welche denen der Auswertung von biometrischen Fotos entsprechen.

Die Auswertung der Strukturinformation bzw. die Ermittlung des mindestens einen Behandlungsparameters kann hierbei mit der Vorrichtung, welche auch den Struktursensor aufweist, durchgeführt werden. Beispielsweise ist der Struktursensor an einem mobilen Gerät, einer Behandlungsvorrichtung wie einer Reinigungsvorrichtung oder einem Behälter wie einer Produktverpackung von Behandlungsmitteln, beispielsweise einer Verpackung eines Reinigungsmittel angeordnet und die gleiche Vorrichtung weist ebenso eine Auswerteeinheit auf, welche die Ermittlung des mindestens einen Behandlungsparameters durchführt oder veranlasst.

Ebenso kann die Auswertung der Strukturinformation bzw. die Ermittlung des mindestens einen Behandlungsparameters von einer weiteren Vorrichtung vorgenommen werden, welche insbesondere über ein Kommunikationssystem mit der Vorrichtung, welche den Struktursensor aufweist, in Verbindung steht. Hierfür kann ein Server vorgesehen sein, welcher die Auswertung ausführt oder weitere Vorrichtungen veranlasst, die Auswertung durchzuführen. Ein solcher Server ist beispielsweise ein Datenbankserver. Beispiele eines Datenbankservers umfassen Microsoft SQL Server, Oracle Server und MySQL Server. Die Server können beispielsweise ein Teil bzw. eine Komponente einer sogenannten Computer Cloud sein, welche Datenverarbeitungsressourcen dynamisch für verschiedene Nutzer in einem Kommunikationssystem bereitstellt. Unter einer Computer Cloud wird insbesondere eine Datenverarbeitungs-Infrastruktur gemäß der Definition des "National Institute for Standards and Technology" (NIST) für den englischen Begriff "Cloud Computing" verstanden. Ein Beispiel einer Computer Cloud ist eine Microsoft Windows Azure Platform.

In einer weiteren Ausgestaltung des Verfahrens gemäß dem ersten Aspekt umfasst das Verfahren weiter ein Beaufschlagen der Textilie mit einer Anregung. Hiermit kann der Ermittlung der Strukturinformation über den Struktursensor eine definierte Anregung zugrunde gelegt werden. Beispielsweise wird die Textilie für ein optisches Sensorelement über eine Lichtquelle mit Strahlung beaufschlagt bzw. beleuchtet, wobei die verwendete Strahlung eine bestimmte Intensität und/oder bestimmte spektrale Verteilung aufweist. Durch ein solches Beleuchten zumindest eines Teils der Textilie wird erreicht, dass unabhängig von den äußeren Bedingungen eine ausreichende Beleuchtung der Textilie insbesondere zur Verwendung eines optischen Sensorelements sichergestellt wird. Dadurch kann ein Ermitteln der Strukturinformation auch bei schlechten äußeren Bedingungen, wie wenig Tageslicht in einem dunklen Raum, ermöglicht werden oder jedenfalls die Qualität der Ermittlung verbessert werden.

Unter einem Beleuchten wird verstanden, dass eine Lichterzeugung mit Hilfe einer künstlichen Lichtquelle erfolgt, sodass insbesondere eine (bessere) Sichtbarmachung der Struktur der Textilie erfolgen kann. Die Beleuchtung erfolgt insbesondere durch Strahlung, welche zumindest teilweise im sichtbaren Bereich des elektromagnetischen Spektrums liegt, beispielsweise mit einer Strahlung dessen Wellenlänge zumindest einen Teil des Wellenlängenbereichs von 380 nm bis 780 nm abdeckt. Ebenso kann die Anregung Anteile im (nahen) Infrarotbereich und/oder im ultravioletten Energiebereich umfassen. Der Wellenlängenbereich kann weiter spezifisch auf einen Struktursensor abgestimmt sein.

Insbesondere für ein akustisches Sensorelement kann eine Beaufschlagung mit Schall bzw. eine Schallquelle vorgesehen sein, ebenfalls mit einer definierten Intensität und Frequenzgang. Die Anregung kann auch eine bestimmte zeitliche Abfolge aufweisen, beispielsweise wird ein Anregungssignal zu einer spezifischen Zeit ausgelöst, weist eine spezifische Dauer auf und/oder umfasst einen bestimmten zeitlichen Ablauf der Intensität und des Frequenzgangs.

Die Dauer der Anregung kann insbesondere relativ kurz, d.h. unterhalb einer Sekunde. Bei einer optischen Anregung wird beispielsweise ein Blitzlicht verwendet. Die Dauer der Anregung kann weiter kleiner als 0,1 Sekunden, insbesondere kleiner als 0,01 Sekunden betragen.

In einer Ausgestaltung der Vorrichtung gemäß dem zweiten Aspekt sind mindestens eine Anregungsvorrichtung und mindestens ein Struktursensor integral an einer Vorrichtung angeordnet und befinden sich somit an dem gleichen Gerät, beispielsweise einem mobilen Gerät. Mit einer solchen kombinierten Anordnung wird die Benutzerfreundlichkeit der Verwendung der Vorrichtung erhöht. Ein mobiles Gerät wie ein Smart-Stift kann vorgesehen sein, welcher an und/oder in der Behandlungsvorrichtung lösbar angeordnet ist und beispielsweise über ein Kabel und/oder über Funk mit der Behandlungsvorrichtung kommunizieren kann. Ein solches mobiles Gerät kann einen oder mehrere Sensoren umfassen.

In einer weiteren Ausgestaltung umfasst das Verfahren weiterhin: Bestimmen eines Nutzerprofils zumindest teilweise basierend auf der Strukturinformation, insbesondere basierend auf einer Mehrzahl von Strukturinformationen, wobei die Ermittlung des mindestens einen Behandlungsparameters zumindest teilweise auf dem Nutzerprofil basiert.

Über die mindestens eine Ausgangsgröße kann somit ein Nutzerprofil erstellt werden, welches an die jeweilige Struktur der Textilie angepasst wird. Insbesondere kann eine Mehrzahl von Behandlungsparametern im Sinne einer Historie von ermittelten Behandlungsparametern in ein Nutzerprofil einfließen, so dass zukünftige Ermittlungen zumindest teilweise auf dem Nutzerprofil basieren können. Damit kann die Ermittlung des mindestens einen Behandlungsparameters adaptiv gestaltet werden und sich über das Nutzerprofil den jeweiligen Anforderungen genauer anpassen. Die Ermittlung des Behandlungsparameters kann insbesondere im Hinblick auf die Abhängigkeit von der Struktur der Textilie genauer durchgeführt werden.

Beispielsweise kann ein Nutzerprofil im Hinblick auf häufig zu behandelnde Strukturen von Textilien erstellt werden.

Denkbar ist es ebenfalls, dass eine Information über die Effektivität der Behandlung in das Nutzerprofil aufgenommen wird. Beispielsweise kann nach einer Reinigungsbehandlung erneut eine Strukturinformation ermittelt werden, um die Wirkung der Reinigungsbehandlung auf die Struktur der Textilie, insbesondere in Hinsicht auf einen entstehenden Verschleiß zu untersuchen. Damit können zukünftige Behandlungen über das Nutzerprofil weiter optimiert werden.

Ebenso kann der Benutzer nach der Behandlung eine Bewertung des mindestens einen Behandlungsparameters, beispielsweise eine Bewertung einer Reinigungsbehandlung vornehmen, welche in das Nutzerprofil eingeht. Damit kann eine persönliche Anpassung der Ermittlung der Behandlungsparameter erreicht werden.

Insbesondere kann das Nutzerprofil auch von weiteren Nutzerprofilen anderer Personen beeinflusst werden. Beispielsweise können Präferenzen und/oder Vergleichswerte mit denen anderer Nutzer abgeglichen werden bzw. als Vorschlag eingebracht werden. Im Rahmen eines solchen Crowdsourcing kann die Auswertung der Strukturinformation weiter optimiert werden.

Ebenfalls ist es möglich, dass das Ermitteln des mindestens einen Behandlungsparameters ein maschinelles Lernen umfasst, insbesondere bei der Verwendung eines Nutzerprofils. So kann das Nutzerprofil beispielsweise zumindest teilweise basierend auf maschinellem Lernen bestimmt werden. Unter einem maschinellen Lernen wird verstanden, dass ein künstliches System (zum Beispiel eine Vorrichtung gemäß dem zweiten Aspekt oder ein System gemäß dem dritten Aspekt) aus Beispielen lernt und diese nach Beendigung der Lernphase verallgemeinern kann. Das heißt, es werden nicht einfach die Beispiele auswendig gelernt, sondern es werden Muster und Gesetzmäßigkeiten in den Lerndaten erkannt. Hierzu können unterschiedliche Ansätze verfolgt werden. Beispielsweise kann ein überwachtes Lernen, ein teilüberwachtes Lernen, ein unüberwachtes Lernen, ein bestärktes Lernen und/oder ein aktives Lernen eingesetzt werden, insbesondere in Verbindung mit *deep* learning-Verfahren. Ein überwachtes Lernen kann beispielsweise mittels eines künstlichen neuronalen Netzes (etwa einem rekurrenten neuronalen Netz) oder mittels einer Support Vector Machine erfolgen. Auch ein unüberwachtes Lernen kann beispielsweise mittels eines künstlichen neuronales Netzes (beispielsweis eines Autoencoders) erfolgen. Als Lerndaten dienen dann beispielsweise insbesondere die mehrmalig erhaltenen und/oder ermittelten Strukturinformation bzw. der mindestens eine Behandlungsparameter.

In einer weiteren Ausgestaltung des Verfahrens ist mindestens eine der Vorrichtungen zur Durchführung des Verfahrens ein mobiles Gerät. Insbesondere kann eine Kommunikation über ein Kommunikationssystem zwischen einem mobilen Gerät, beispielsweise einem Smartphone, Laptop, Tablet, Wearable, Smartwatch, Smart-Stift oder einer Kamera, und mindestens einer weiteren Vorrichtung vorgenommen werden, beispielsweise einer Reinigungsvorrichtung, einer Vorrichtung für eine Endbehandlung wie einer Bügelvorrichtung, und/oder einem Struktursensor. Eine der Vorrichtungen kann auch ein Reinigungsroboter sein. Gemäß einer Ausgestaltung umfasst die Vorrichtung gemäß dem zweiten Aspekt eine Kommunikationsschnittstelle. Beispielsweise ist die Kommunikationsschnittstelle für eine drahtgebundene oder drahtlose Kommunikation eingerichtet. Beispielsweise ist die Kommunikationsschnittstelle eine Netzwerkschnittstelle. Die Kommunikationsschnittstelle ist bevorzugt dazu eingerichtet mit einem Kommunikationssystem zu kommunizieren. Beispiele für ein Kommunikationssystem sind ein lokales Netzwerk (LAN), ein großräumiges Netzwerk (WAN), ein drahtloses Netzwerk (beispielsweise gemäß dem IEEE-802.11-Standard, dem Bluetooth (LE)-Standard und/oder dem NFC-Standard), ein drahtgebundenes Netzwerk, ein Mobilfunknetzwerk, ein Telefonnetzwerk und/oder das Internet. Ein Kommunikationssystem kann die Kommunikation mit einem externen Computer umfassen, beispielsweise über eine Internetverbindung.

Gemäß dem zweiten Aspekt der Erfindung wird auch eine alternative Vorrichtung beschrieben, umfassend zumindest einen Prozessor und zumindest einen Speicher mit Computerprogrammcode, wobei der zumindest eine Speicher und der Computerprogrammcode dazu eingerichtet sind, mit dem zumindest einen Prozessor zumindest ein Verfahren gemäß dem ersten Aspekt auszuführen und/oder zu steuern. Unter einem Prozessor soll zum Beispiel eine Kontrolleinheit, ein Mikroprozessor, eine Mikrokontrolleinheit wie ein Mikrocontroller, ein digitaler Signalprozessor (DSP), eine anwendungsspezifische Integrierte Schaltung (ASIC) oder ein Field Programmable Gate Arrays (FPGA) verstanden werden.

Zum Beispiel umfasst eine beispielhafte Vorrichtung ferner Mittel zum Speichern von Informationen wie einen Programmspeicher und/oder einen Hauptspeicher. Zum Beispiel umfasst eine beispielhafte erfindungsgemäße Vorrichtung ferner jeweils Mittel zum Empfangen und/oder Senden von Informationen über ein Netzwerk wie eine Netzwerkschnittstelle. Zum Beispiel sind beispielhafte erfindungsgemäße Vorrichtungen über ein oder mehrere Netzwerke miteinander verbunden und/oder verbindbar.

Eine beispielhafte Vorrichtung gemäß dem zweiten Aspekt ist oder umfasst etwa eine Datenverarbeitungsanlage, die softwaremäßig und/oder hardwaremäßig eingerichtet ist, um die jeweiligen Schritte eines beispielhaften Verfahrens gemäß dem ersten Aspekt ausführen zu können. Beispiele für eine Datenverarbeitungsanlage sind ein Computer, ein Desktop-Computer, ein Server, ein Thinclient und/oder ein tragbarer Computer (Mobilgerät), wie etwa ein Laptop-Computer, ein Tablet-Computer, ein Wearable, ein persönlicher digitaler Assistent oder ein Smartphone.

Gemäß dem zweiten Aspekt der Erfindung wird auch ein Computerprogramm beschrieben, das Programmanweisungen umfasst, die einen Prozessor zur Ausführung und/oder Steuerung eines Verfahrens gemäß dem ersten Aspekt veranlassen, wenn das Computerprogramm auf dem Prozessor läuft. Ein beispielhaftes Programm gemäß der Erfindung kann in oder auf einem computerlesbaren Speichermedium gespeichert sein, welches eines oder mehrere Programme enthält.

Gemäß dem zweiten Aspekt der Erfindung wird auch ein computerlesbares Speichermedium beschrieben, welches ein Computerprogramm gemäß dem zweiten Aspekt enthält. Ein computerlesbares Speichermedium kann z.B. als magnetisches, elektrisches, elektromagnetisches, optisches und/oder andersartiges Speichermedium ausgebildet sein. Ein solches computerlesbares Speichermedium ist vorzugsweise gegenständlich (also "berührbar"), zum Beispiel ist es als Datenträgervorrichtung ausgebildet. Eine solche Datenträgervorrichtung ist beispielsweise tragbar oder in einer Vorrichtung fest installiert. Beispiele für eine solche Datenträgervorrichtung sind flüchtige oder nicht-flüchtige Speicher mit wahlfreiem-Zugriff (RAM) wie z.B. NOR-Flash-Speicher oder mit sequentiellen-Zugriff wie NAND-Flash-Speicher und/oder Speicher mit Nur-Lese-Zugriff (ROM) oder Schreib-Lese-Zugriff. Computerlesbar soll zum Beispiel so verstanden werden, dass das Speichermedium von einem Computer bzw. einer Datenverarbeitungsanlage (aus)gelesen und/oder beschrieben werden kann, beispielsweise von einem Prozessor.

Gemäß einem dritten Aspekt der Erfindung wird auch ein System beschrieben, umfassend mehrere Vorrichtungen, insbesondere ein mobiles Gerät und eine Behandlungsvorrichtung, welche zusammen ein Verfahren gemäß dem ersten Aspekt durchführen.

Ein beispielhaftes System gemäß dem dritten Aspekt umfasst eine beispielhafte Reinigungsvorrichtung und zusätzlich eine weitere Vorrichtung, beispielsweise ein mobiles Gerät oder einen Server zur Durchführung eines beispielhaften Verfahrens gemäß dem ersten Aspekt.

Insbesondere kann das System gemäß dem dritten Aspekt auch weiter mindestens eine Textilie bzw. einen Satz von Textilien umfassen.

Die zuvor in dieser Beschreibung beschriebenen beispielhaften Ausgestaltungen der vorliegenden Erfindung sollen auch in allen Kombinationen miteinander offenbart verstanden werden. Insbesondere sollen beispielhafte Ausgestaltungen in Bezug auf die unterschiedlichen Aspekten offenbart verstanden werden.

Insbesondere sollen durch die vorherige oder folgende Beschreibung von Verfahrensschritten gemäß bevorzugter Ausführungsformen eines Verfahrens auch entsprechende Mittel zur Durchführung der Verfahrensschritte durch bevorzugte Ausführungsformen einer Vorrichtung offenbart sein. Ebenfalls soll durch die Offenbarung von Mitteln einer Vorrichtung zur Durchführung eines Verfahrensschrittes auch der entsprechende Verfahrensschritt offenbart sein.

Weitere vorteilhafte beispielhafte Ausgestaltungen der Erfindung sind der folgenden detaillierten Beschreibung einiger beispielhafter Ausführungsformen der vorliegenden Erfindung, insbesondere in Verbindung mit den Figuren, zu entnehmen. Die Figuren sollen jedoch nur dem Zwecke der Verdeutlichung, nicht aber zur Bestimmung des Schutzbereiches der Erfindung dienen. Die Figuren sind nicht maßstabsgetreu und sollen lediglich das allgemeine Konzept der vorliegenden Erfindung beispielhaft widerspiegeln. Insbesondere sollen Merkmale, die in den Figuren enthalten sind, keineswegs als notwendiger Bestandteil der vorliegenden Erfindung erachtet werden.

### Kurze Beschreibung der Figuren

In der Zeichnung zeigt
- Fig. 1: ein Ablaufdiagramm eines Ausführungsbeispiels eines Verfahrens;
- Fig. 2: eine schematische Darstellung eines Ausführungsbeispiels einer Vorrichtung;
- Fig. 3: ein Blockdiagramm eines Ausführungsbeispiels einer Vorrichtung; und
- Fig. 4: unterschiedliche Ausführungsbeispiele eines Speichermediums.

### Detaillierte Beschreibung einiger beispielhafter Ausführungsformen der Erfindung

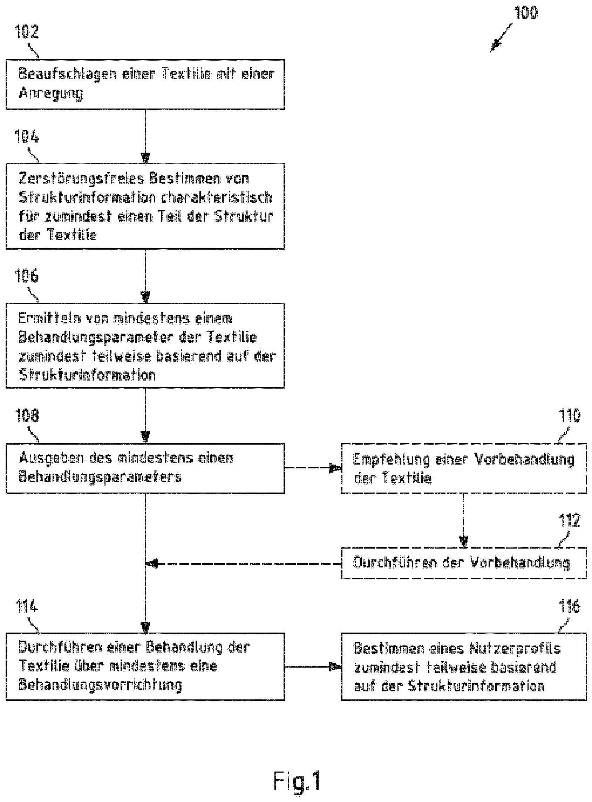

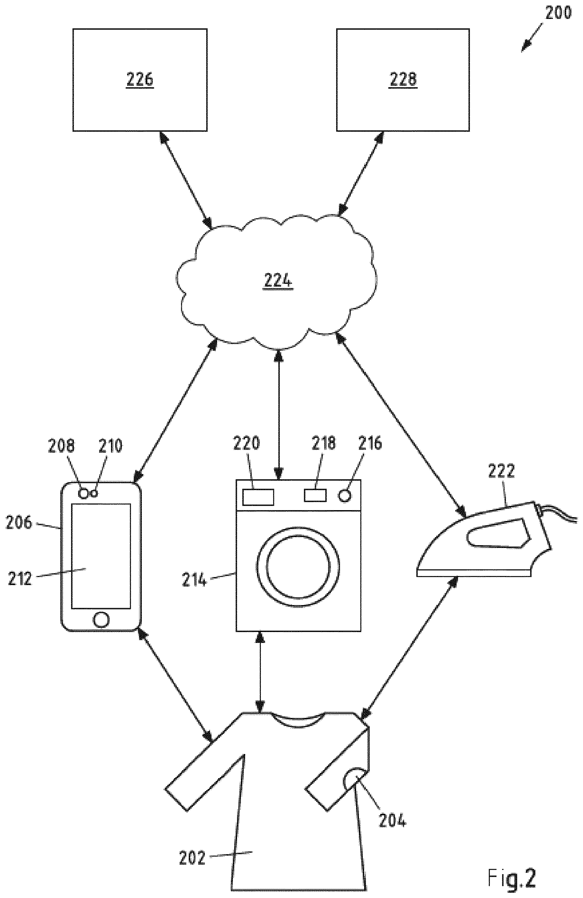

Fig.1 zeigt ein Ablaufdiagramm 100 eines Ausführungsbeispiels eines Verfahrens gemäß dem ersten Aspekt, welches durch eine Vorrichtung, beispielsweise eine der Vorrichtungen aus Fig. 2 durchgeführt werden kann.

In Aktion 102 wird eine Textilie, beispielsweise ein Kleidungsstück, mit einer Anregung beaufschlagt. Insbesondere wird eine optische Anregung der Textilie durch eine Beaufschlagung mit Strahlung bzw. eine Beleuchtung durchgeführt. Hierbei kann eine Lichterzeugung mit Hilfe einer künstlichen Lichtquelle erfolgen, sodass insbesondere eine bessere Sichtbarmachung der Struktur der Textilie bewirkt wird, beispielsweise mit einem Blitzlicht. Ebenso kann eine optische Anregung über eine natürliche Lichtquelle, beispielsweise durch Tageslicht erfolgen. Weiter möglich ist eine akustische Anregung der Textilie in Form einer Beaufschlagung mit Schall. Insbesondere basierend auf dieser Anregung kann eine zerstörungsfreie Bestimmung von Strukturinformation in Aktion 104 vorgenommen werden. Die Strukturinformation ist dabei charakteristisch für zumindest einen Teil der Struktur der Textilie.

Beispielsweise wird bei einer Beleuchtung der Textilie die von der Oberfläche der Textilie resultierende Strahlung über ein optisches Sensorelement aufgenommen. Hiermit kann eine Bildinformation mit einer dreidimensionalen Auflösung erhalten werden, welche charakteristisch für die Struktur der Textilie ist.

Ebenso kann insbesondere mit einer akustischen Anregung über mindestens ein akustisches Sensorelement eine Strukturinformation der Textilie erhalten werden. Insbesondere wird die Schallschnelle v, Schallfluss q, Schalldruck *p*, Schallimpedanz Z, Schallgeschwindigkeit c, Auslenkung ξ des Schalls oder eine Kombination hiervon erfasst, um für die Struktur der Textilie charakteristische Strukturinformation zu erhalten. Insbesondere werden richtungsabhängige Größen auch richtungsabhängig erfasst.

Die Strukturinformation kann hierbei indikativ für Materialstruktur, Materialart, Materialverteilung und Materialverschleiß der Textilie sein. Beispielsweise wird über die Strukturinformation, nämlich die dreidimensionale Bildinformation, die Höhe, die Form und Anzahl von Pillings an der Textilie erfasst. Weiter können verschiedene Materialienarten, beispielsweise Wolle, Baumwolle und Kunstfasern, oder Materialstrukturen wie Gewebe oder Vlies von der Strukturinformation charakterisiert werden. Ebenso ist es möglich, dass die Strukturinformation indikativ für das Vorhandensein und von Verschlussmitteln, etwa Reißverschlüssen oder Knöpfen, von Beschichtungsmaterial und von Applikationen auf der Textilie ist.

Zumindest teilweise basierend auf der Strukturinformation wird in Aktion 106 mindestens ein Behandlungsparameter der Textilie ermittelt. Der Behandlungsparameter kann indikativ für eine Empfehlung einer Vorbehandlung sein, beispielsweise einer Auftragung eines Vorbehandlungsmittels. Ebenso kann beispielsweise mit einer für eine Applikation auf der Textilie charakteristischen Strukturinformation ein Drehen der Textilie "auf links" oder eine empfohlene Anordnung der Textilie in einem Wäschebeutel angezeigt werden.

Der Behandlungsparameter kann weiter indikativ für eine Empfehlung einer Reinigungsbehandlung sein, etwa für eine Reinigungsmittelart, eine Reinigungsmittelmenge, eine Reinigungstemperatur, einen Reinigungsvorrichtungstyp und Einstellungen einer Reinigungsvorrichtung wie einer Waschmaschine. Ist die Strukturinformation charakteristisch für ein Gewebe aus Wolle in der Textilie, kann beispielsweise ein Wollwaschprogramm bei niedrigen Temperaturen mit einem speziellen Wollwaschmittel über die Behandlungsparameter angegeben werden.

Ebenso kann der Behandlungsparameter indikativ für eine Endbehandlung der Textilie sein. Beispielsweise zeigt der mindestens eine Behandlungsparameter die Temperatur und ein Programm für ein Bügeln oder Trocknen der Textilie an.

Der mindestens eine Behandlungsparameter wird in Aktion 108 ausgegeben und beispielsweise auf einer Anzeigevorrichtung zur Verfügung gestellt, womit dem Benutzer die Empfehlung über die Behandlungsparameter der Textilie zugänglich gemacht wird. Dem Benutzer kann beispielsweise die Materialart und der Materialverschleiß angezeigt werden sowie Sätze von

Behandlungsparametern für eine Vorbehandlung, einer Reinigungsbehandlung und einer Endbehandlung. Für den Benutzer wird damit die Identifikation der Struktur der Textilie sowie die Ermittlung der optimalen Behandlung der Textilie erleichtert bzw. ermöglicht. Der Benutzer kann beispielsweise im Anschluss entscheiden, ob die Behandlungsparameter wie empfohlen übernommen werden sollen und eine Behandlung durchführen.

Wird beispielsweise in Aktion 110 eine Vorbehandlung empfohlen, kann eine automatische oder manuelle Durchführung 112 der Vorbehandlung vorgenommen werden. Beispielsweise dreht der Benutzer die Textilie "auf links", wenn Applikationen an der Textilie angezeigt werden oder schließt einen Reißverschluss.

In Aktion 114 wird eine Behandlung durchgeführt, beispielsweise eine Reinigungsbehandlung. Insbesondere werden die Behandlungsparametern an mindestens eine Behandlungsvorrichtung ausgegeben und dienen einer Voreinstellung. Beispielsweise übernimmt eine Waschmaschine eine Reinigungstemperatur und ein Reinigungsprogramm als Voreinstellung. Der Benutzer muss dann die Waschmaschine lediglich starten. Ebenso kann mindestens eine Behandlungsvorrichtung automatisch eine Behandlung auf Grundlage der ausgegebenen Behandlungsparameter durchführen. Eine Reinigungsvorrichtung kann beispielsweise auch über eine Dosiervorrichtung verfügen, welche automatisch eine Reinigungsmittelart und eine Reinigungsmittelmenge entsprechend den ausgegebenen Behandlungsparametern bereitstellt.

Ebenso kann beispielsweise eine Behandlung in Aktion 114 über eine Bügelvorrichtung vorgenommen werden. Hierbei wird insbesondere die Temperatur der Bügelvorrichtung entsprechend dem Behandlungsparametern reguliert. Beispielsweise wird die Strukturinformation kontinuierlich aufgenommen, so dass die Bügelvorrichtung bei der Bewegung über die Textilie den Anforderungen des jeweiligen Teilbereichs der Textilie entsprechend betrieben wird.

Zusätzlich kann in Aktion 116 ein Nutzerprofil bestimmt werden, welches zumindest teilweise auf dem Behandlungsparameter basiert. Damit kann die Ermittlung des mindestens einen Behandlungsparameters adaptiv gestaltet werden und sich über das Nutzerprofil den jeweiligen Anforderungen genauer anpassen.

Fig. 2 zeigt ein Ausführungsbeispiel von Vorrichtungen gemäß dem zweiten Aspekt bzw. eines Systems 200 gemäß dem dritten Aspekt.

Eine Textilie 202 in Form eines Kleidungsstücks weist eine bestimmte Struktur auf, beispielsweise besteht die Textilie 202 unter anderem aus einem Gewebe aus Wolle, Baumwolle oder Kunstfasern. Beispielsweise befindet sich auf dem Gewebe der Textilie 202 eine Applikation 204.

Die Struktur der Textilie 202 kann dabei eine bestimmte Form einer Behandlung, beispielsweise eine bestimmte Form der Reinigung oder des Bügelns erfordern.

Mit einem mobilen Gerät 206, hier einem Smartphone, kann beispielsweise eine Strukturinformation der Textilie 202 ermittelt werden. Hierfür ist ein Struktursensor 208 vorgesehen, insbesondere ein optischer Sensor bzw. ein kameraartiges Element. Der Struktursensor 208 erfasst beispielsweise die von der Oberfläche der Textilie ausgehende Strahlung bzw. eine Bildinformation. Zusätzlich ist eine Strahlungsquelle 210 vorgesehen, welche zur Beleuchtung der Oberfläche der Struktur der Textilie 202 dient. Das mobile Gerät 206 verfügt zudem über ein Anzeigeelement 212.

Ebenso kann die Bestimmung der Strukturinformation über einen Struktursensor (nicht gezeigt) in einer Reinigungsvorrichtung 214, hier einer Waschmaschine vorgesehen sein. Der Struktursensor umfasst beispielsweise ein akustisches Sensorelement, wobei die Textilie über eine Beaufschlagung mit Schall angeregt wird und der Struktursensor die Reaktion der Struktur der Textilie auf diese Anregung aufnimmt. Die Reinigungsvorrichtung 214 weist weiter ein Bedienelement 216 zur manuellen Bedienung, ein Anzeigeelement 218 sowie eine Dosiervorrichtung 220 für ein Reinigungsmittel auf.

Weiter ist in Fig. 2 als Behandlungsvorrichtung 222 eine Bügelvorrichtung dargestellt, welche einer Endbehandlung bzw. einer Glättung der Textilie dient. Die Behandlungsvorrichtung 222 kann ebenfalls einen Struktursensor aufweisen (nicht gezeigt).

Die ermittelte Strukturinformation wird von einem Kommunikationssystem 224 erhalten, welche in Verbindung mit dem mobilen Gerät 206, der Reinigungsvorrichtung 214 und der Behandlungsvorrichtung 222 steht. In Verbindung mit dem Kommunikationssystem 224 steht weiter eine Ermittlungsvorrichtung 226, welche dafür eingerichtet ist, mindestens einen von der Strukturinformation der Textilie 202 abhängigen Behandlungsparameter zu ermitteln. Die Ermittlungsvorrichtung 226 kann als separate Vorrichtung ausgebildet sein oder auch beispielsweise in dem mobilen Gerät 206, der Reinigungsvorrichtung 214 und der Behandlungsvorrichtung 222 integriert sein.

Die Ermittlung des mindestens einen Behandlungsparameters umfasst beispielsweise die Verwendung von mindestens einem Bearbeitungsalgorithmus, beispielsweise einem Konvertierungsalgorithmus. Beispielsweise wird die Bildinformation des optischen Struktursensors 208 einer Bildauswertung unterzogen, nämlich einer dreidimensionalen Bildauswertung. Die Ermittlungsvorrichtung 226 kann auch über das Kommunikationssystem 224 in Verbindung mit mindestens einem weiteren Computer 228 stehen. Der Computer 228 kann beispielsweise dynamisch zusätzliche Rechenleistung zur Ermittlung des mindestens einen Behandlungsparameters bereitstellen und auch eine Datenbank mit Vergleichswerten aufweisen.

Der mindestens eine Behandlungsparameter wird ausgegeben, insbesondere auf der Anzeigevorrichtung 212 des mobilen Geräts 206 oder der Anzeigevorrichtung 218 der Reinigungsvorrichtung 214. Der Benutzer kann dann entsprechend den Behandlungsparametern bzw. der identifizierten Struktur der Textilie 202 eine Behandlung auswählen. Beispielsweise übernimmt die Reinigungsvorrichtung 214 eine Reinigungstemperatur und ein Reinigungsprogramm als Voreinstellung. Die Dosiervorrichtung 220 kann automatisch eine Reinigungsmittelart und eine Reinigungsmittelmenge entsprechend den ausgegebenen Behandlungsparametern bereitstellen. Der Benutzer muss dann lediglich die Reinigungsvorrichtung 214 über das Bedienelement 216 starten, um eine optimale Reinigung der Textilie 202 durchführen zu lassen.

Ebenso kann beispielsweise die Temperatur der Behandlungsvorrichtung 222 bei einem Bügeln der Textilie 202 über die Vorrichtung 200 reguliert werden, so dass eine besonders schonende Behandlung der Textilie 202 gewährleistet ist. Dabei kann der Struktursensor der Behandlungsvorrichtung 222 kontinuierlich während der Behandlung Strukturinformation ermitteln, so dass beispielsweise die Temperatur der Behandlungsvorrichtung 222 der Struktur des gerade behandelten Teilabschnitts der Textilie entsprechend eingestellt werden kann.

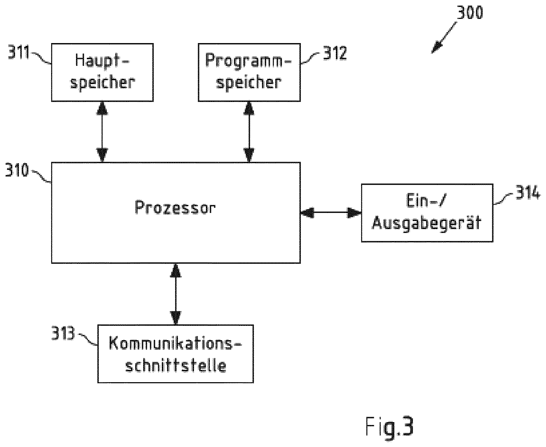

Fig. 3 zeigt ein Blockdiagramm eines Ausführungsbeispiels einer Vorrichtung 300, welche insbesondere ein beispielhaftes Verfahren gemäß dem ersten Aspekt ausführen kann. Die Vorrichtung 300 ist beispielsweise eine Vorrichtung gemäß dem zweiten oder ein System gemäß dem dritten Aspekt.

Die Vorrichtung 300 kann insofern beispielsweise ein Computer, ein Desktop-Computer, ein Server, ein Thinclient oder ein tragbarer Computer (Mobilgerät), wie etwa ein Laptop-Computer, ein Tablet-Computer, ein persönlicher digitaler Assistent (PDA) oder ein Smartphone sein. Die Vorrichtung kann beispielsweise die Funktion eines Servers oder eines Clients erfüllen.

Prozessor 310 der Vorrichtung 300 ist insbesondere als Mikroprozessor, Mikrokontrolleinheit, Mikrocontroller, digitaler Signalprozessor (DSP), Anwendungsspezifische Integrierte Schaltung (ASIC) oder Field Programmable Gate Array (FPGA) ausgebildet.

Prozessor 310 führt Programmanweisungen aus, die in Programmspeicher 312 gespeichert sind, und speichert beispielsweise Zwischenergebnisse oder ähnliches in Arbeits- oder Hauptspeicher 311. Zum Beispiel ist Programmspeicher 312 ein nicht-flüchtiger Speicher wie ein Flash-Speicher, ein Magnetspeicher, ein EEPROM-Speicher (elektrisch löschbarer programmierbarer Nur-Lese-Speicher) und/oder ein optischer Speicher. Hauptspeicher 311 ist zum Beispiel ein flüchtiger oder nicht-flüchtiger Speicher, insbesondere ein Speicher mit wahlfreiem-Zugriff (RAM) wie ein statischer RAM-Speicher (SRAM), ein dynamischer RAM-Speicher (DRAM), ein ferroelektrischer RAM-Speicher (FeRAM) und/oder ein magnetischer RAM-Speicher (MRAM).

Programmspeicher 312 ist vorzugsweise ein lokaler mit der Vorrichtung 400 fest verbundener Datenträger. Mit der Vorrichtung 300 fest verbundene Datenträger sind beispielsweise Festplatten, die in die Vorrichtung 300 eingebaut sind. Alternativ kann der Datenträger beispielsweise auch ein mit der Vorrichtung 300 trennbar verbindbarer Datenträger sein wie ein Speicher-Stick, ein Wechseldatenträger, eine tragbare Festplatte, eine CD, eine DVD und/oder eine Diskette.

Programmspeicher 312 enthält beispielsweise das Betriebssystem von der Vorrichtung 300, das beim Starten der Vorrichtung 300 zumindest teilweise in Hauptspeicher 311 geladen und vom Prozessor 310 ausgeführt wird. Insbesondere wird beim Starten von Vorrichtung 300 zumindest ein Teil des Kerns des Betriebssystems in den Hauptspeicher 311 geladen und von Prozessor 310 ausgeführt. Das Betriebssystem von Vorrichtung 300 ist beispielsweise ein Windows -, UNIX-, Linux-, Android-, Apple iOS- und/oder MAC-Betriebssystem.

Das Betriebssystem ermöglicht insbesondere die Verwendung der Vorrichtung 300 zur Datenverarbeitung. Es verwaltet beispielsweise Betriebsmittel wie Hauptspeicher 311 und Programmspeicher 312, Netzwerkschnittstelle 313, Ein- und Ausgabegerät 314, stellt unter anderem durch Programmierschnittstellen anderen Programmen grundlegende Funktionen zur Verfügung und steuert die Ausführung von Programmen.

Prozessor 310 steuert die Kommunikationsschnittstelle 313, welche beispielsweise eine Netzwerkschnittstelle sein kann und als Netzwerkkarte, Netzwerkmodul und/oder Modem ausgebildet sein kann. Die Kommunikationsschnittstelle 313 ist insbesondere dazu eingerichtet, eine Verbindung der Vorrichtung 300 mit anderen Vorrichtungen, insbesondere über ein (drahtloses) Kommunikationssystem, beispielsweise ein Netzwerk, herzustellen und mit diesen zu kommunizieren. Die Kommunikationsschnittstelle 313 kann beispielsweise Daten (über das Kommunikationssystem) empfangen und an Prozessor 310 weiterleiten und/oder Daten von Prozessor 310 empfangen und (über das Kommunikationssystem) senden. Beispiele für ein Kommunikationssystem sind ein lokales Netzwerk (LAN), ein großräumiges Netzwerk (WAN), ein drahtloses Netzwerk (beispielsweise gemäß dem IEEE-802.11-Standard, dem Bluetooth (LE)-Standard und/oder dem NFC-Standard), ein drahtgebundenes Netzwerk, ein Mobilfunknetzwerk, ein Telefonnetzwerk und/oder das Internet.

Des Weiteren kann Prozessor 310 zumindest ein Ein-/Ausgabegerät 314 steuern. Ein-/Ausgabegerät 314 ist beispielsweise eine Tastatur, eine Maus, eine Anzeigeeinheit, ein Mikrofon, eine berührungsempfindliche Anzeigeeinheit, ein Lautsprecher, ein Lesegerät, ein Laufwerk und/oder eine Kamera. Ein-/Ausgabegerät 314 kann beispielsweise Eingaben eines Benutzers aufnehmen und an Prozessor 310 weiterleiten und/oder Informationen für den Benutzer von Prozessor 310 empfangen und ausgeben.

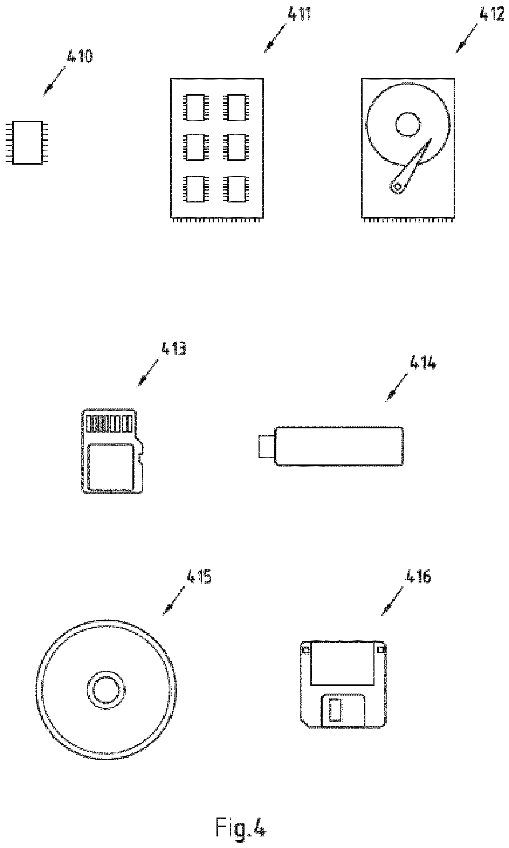

Fig.4 zeigt schließlich unterschiedliche Ausführungsbeispiele von Speichermedien, auf denen ein Ausführungsbeispiel eines erfindungsgemäßen Computerprogrammes gespeichert sein kann. Das Speichermedium kann beispielsweise ein magnetisches, elektrisches, optisches und/oder andersartiges Speichermedium sein. Das Speichermedium kann beispielsweise Teil eines Prozessors (z.B. des Prozessor 310 der Fig. 3) sein, beispielsweise ein (nicht-flüchtiger oder flüchtiger) Programmspeicher des Prozessors oder ein Teil davon (wie Programmspeicher 312 in Fig. 3). Ausführungsbeispiele eines Speichermediums sind ein Flash-Speicher 410, eine SSD-Festplatte 411, eine magnetische Festplatte 412, eine Speicherkarte 413, ein Memory Stick 414 (z.B. ein USB-Stick), eine CD-ROM oder DVD 415 oder eine Diskette 416.

Die in dieser Spezifikation beschriebenen Ausführungsbeispiele der vorliegenden Erfindung und die diesbezüglich jeweils angeführten optionalen Merkmale und Eigenschaften sollen auch in allen Kombinationen miteinander offenbart verstanden werden. Insbesondere soll auch die Beschreibung eines von einem Ausführungsbeispiel umfassten Merkmals - sofern nicht explizit gegenteilig erklärt - vorliegend nicht so verstanden werden, dass das Merkmal für die Funktion des Ausführungsbeispiels unerlässlich oder wesentlich ist.

Die Verfahrensschritte können auf verschiedene Art und Weise implementiert werden, so ist eine Implementierung in Software (durch Programmanweisungen), Hardware oder eine Kombination von beidem zur Implementierung der Verfahrensschritte denkbar.

In den Patentansprüchen verwendete Begriffe wie "umfassen", "aufweisen", "beinhalten", "enthalten" und dergleichen schließen weitere Elemente oder Schritte nicht aus. Unter die Formulierung "zumindest teilweise" fallen sowohl der Fall "teilweise" als auch der Fall "vollständig". Die Formulierung "und/oder" soll dahingehend verstanden werden, dass sowohl die Alternative als auch die Kombination offenbart sein soll, also "A und/oder B" bedeutet "(A) oder (B) oder (A und B)". Die Verwendung des unbestimmten Artikels schließt eine Mehrzahl nicht aus. Eine einzelne Vorrichtung kann die Funktionen mehrerer in den Patentansprüchen genannten Einheiten bzw.

Vorrichtungen ausführen. In den Patentansprüchen angegebene Bezugszeichen sind nicht als Beschränkungen der eingesetzten Mittel und Schritte anzusehen.

## Patentansprüche

1. Verfahren durchgeführt von einer oder mehreren Vorrichtungen, umfassend:
- zerstörungsfreies Bestimmen von Strukturinformation charakteristisch für zumindest einen Teil der Struktur einer Textilie (202);
- Ermitteln von mindestens einem Behandlungsparameter der Textilie (202) zumindest teilweise basierend auf der Strukturinformation; und
- Ausgeben oder Auslösen eines Ausgebens des mindestens einen Behandlungsparameters,
**dadurch gekennzeichnet dass** zur Bestimmung der Strukturinformation mindestens ein optisches Sensorelement verwendet wird, wobei das mindestens eine optische Sensorelement eine dreidimensionale räumliche Auflösung bereitstellt, und
wobei die Strukturinformation indikativ für die Höhe, die Form und/oder Anzahl von Pillings an der Textilie (202) ist, und/oder
wobei die Strukturinformation indikativ für das Vorhandensein und/oder Art von Verschlussmitteln, von Beschichtungsmaterial und/oder von Applikationen (204) in, an und/oder auf der Textilie (202) ist.

2. Verfahren nach Anspruch 1, wobei die Strukturinformation ferner indikativ für Materialstruktur, Materialart, Materialverteilung, Materialverschleiß der Textilie (202) oder eine Kombination hiervon ist.

3. Verfahren nach Anspruch 1 oder 2, wobei der mindestens eine Behandlungsparameter indikativ für eine Empfehlung einer Vorbehandlung, einer Reinigungsbehandlung und/oder einer Endbehandlung der Textilie (202) ist, und/oder
wobei der mindestens eine Behandlungsparameter indikativ für eine Reinigungsmittelart, eine Reinigungsmittelmenge, eine Reinigungstemperatur, einen Reinigungsvorrichtungstyp, Einstellungen einer Reinigungsvorrichtung (214) oder eine Kombination hiervon ist.

4. Verfahren nach einem der Ansprüche 1 bis 3, das Verfahren weiterhin umfassend:
- Durchführen oder Veranlassung der Durchführung einer Behandlung der Textilie (202) entsprechend dem mindestens einen ermittelten Behandlungsparameter über mindestens eine Behandlungsvorrichtung (222), insbesondere eine Reinigungsvorrichtung (214).

5. Verfahren nach einem der Ansprüche 1 bis 4, wobei die Strukturinformation vor, während und/oder nach einer Behandlung der Textilie (202) bestimmt wird.

6. Verfahren nach einem der Ansprüche 1 bis 5, wobei zur Bestimmung der Strukturinformation der Textilie (202) mindestens ein optisches Sensorelement verwendet wird.

7. Verfahren nach Anspruch 6, wobei das optische Sensorelement eine dreidimensionale räumliche Auflösung bereitstellt.

8. Verfahren nach Anspruch 6 oder 7, wobei das mindestens eine optische Sensorelement mindestens ein kameraartiges Element (208) umfasst und eine Bildinformation der Textilie (202) bereitstellt.

9. Verfahren nach Anspruch 8, wobei die Bildinformation mindestens zwei Einzelbilder der Textilie (202) umfasst.

10. Verfahren nach einem der Ansprüche 1 bis 9, wobei die Strukturinformation der Textilie (202) eine Bildinformation einer Beschriftung einer Markierung der Textilie (202) umfasst und insbesondere eine Texterkennung und/oder Klassifizierung der Beschriftung vorgenommen wird.

11. Verfahren nach einem der Ansprüche 1 bis 10, wobei zur Bestimmung der Strukturinformation der Textilie (202) mindestens ein akustisches Sensorelement verwendet wird.

12. Verfahren nach einem der Ansprüche 1 bis 11, das Verfahren weiter umfassend:
- Beaufschlagen der Textilie (202) mit einer Anregung, insbesondere Beaufschlagung mit Strahlung und/oder Schall.

13. Verfahren nach einem der Ansprüche 1 bis 12, das Verfahren weiterhin umfassend:
- Bestimmen eines Nutzerprofils zumindest teilweise basierend auf der Strukturinformation, insbesondere basierend auf einer Mehrzahl von Strukturinformationen,
- wobei die Ermittlung des mindestens einen Behandlungsparameters zumindest teilweise auf dem Nutzerprofil basiert.

14. Vorrichtung, wobei die Vorrichtung ein optisches Sensorelement beinhaltet welches dazu eingerichtet ist, eine dreidimensionale räumliche Auflösung bereitzustellen, und wobei die Vorrichtung weiterhin Mittel beinhaltet die dazu eingerichtet sind, alle Schritte eines der Verfahren nach einem der Ansprüche 1 bis 13 durchzuführen und/oder zu steuern.

## Claims

1. A method carried out by one or more devices, comprising:
- non-destructively determining structural information characteristic for at least part of the structure of a textile (202);
- ascertaining at least one treatment parameter of the textile (202) based at least in part on the structural information; and
- outputting or triggering the output of the at least one treatment parameter,
**characterized in that** at least one optical sensor element is used to determine the structural information, the at least one optical sensor element providing a three-dimensional spatial resolution, and
the structural information being indicative of the height, the shape and/or amount of pilling on the textile (202), and/or
the structural information being indicative of the presence and/or type of closure means, coating material, and/or applications (204) in and/or on the textile (202).

2. The method according to claim 1, wherein the structural information is further indicative of material structure, material type, material distribution, material wear of the textile (202), or a combination thereof.

3. The method according to claim 1 or 2, wherein the at least one treatment parameter is indicative of a recommendation of a pretreatment, a cleaning treatment, and/or an end treatment of the textile (202), and/or
wherein the at least one treatment parameter is indicative of a cleaning agent type, a cleaning agent amount, a cleaning temperature, a cleaning device type, settings of a cleaning device (214), or a combination thereof.

4. The method according to one of claims 1 to 3, the method further comprising:
- carrying out a treatment of the textile (202) or causing said treatment to be carried out according to the at least one ascertained treatment parameter by means of at least one treatment device (222); in particular a cleaning device (214).

5. The method according to one of claims 1 to 4, wherein the structural information is determined before, during and/or after a treatment of the textile (202).

6. The method according to one of claims 1 to 5, wherein at least one optical sensor element is used to determine the structural information of the textile (202).

7. The method according to claim 6, wherein the optical sensor element provides a three-dimensional spatial resolution.

8. The method according to claim 6 or 7, wherein the at least one optical sensor element comprises at least one camera-like element (208) and provides image information of the textile (202).

9. The method according to claim 8, wherein the image information comprises at least two individual images of the textile (202).

10. The method according to one of claims 1 to 9, wherein the structural information of the textile (202) comprises image information of a labeling of a marking of the textile (202), and in particular text recognition and/or classification of the labeling is carried out.

11. The method according to one of claims 1 to 10, wherein at least one acoustic sensor element is used to determine the structural information of the textile (202).

12. The method according to one of claims 1 to 11, the method further comprising:
- applying an excitation, in particular applying radiation and/or sound, to the textile (202).

13. The method according to one of claims 1 to 12, the method further comprising:
- determining a user profile based at least in part on the structural information, in particular based on a plurality of items of structural information,
- wherein ascertaining the at least one treatment parameter is based at least in part on the user profile.

14. A device, wherein the device contains an optical sensor element which is configured to provide a three-dimensional spatial resolution, and wherein the device further contains means which are configured to carry out and/or control all the steps of one of the methods according to one of claims 1 to 13.

## Revendications

1. Procédé mis en oeuvre par un ou plusieurs dispositifs, comprenant :
- la détermination de manière non destructive d'une information de structure caractéristique d'au moins une partie de la structure d'un textile (202) ;
- la définition d'au moins un paramètre de traitement du textile (202) au moins en partie sur la base de l'information de structure ; et
- la sortie ou le déclenchement d'une sortie de l'au moins un paramètre de traitement,
**caractérisé en ce que,** pour la détermination de l'information de structure, au moins un élément formant capteur optique est utilisé, dans lequel l'au moins un élément formant capteur optique fournit une résolution spatiale tridimensionnelle, et
dans lequel l'information de structure indique la hauteur, la forme et/ou le nombre de bouloches sur le textile (202), et/ou
dans lequel l'information de structure indique la présence et/ou le type de moyens de fermeture, de matériau de revêtement et/ou d'appliques (204) dans le textile, au niveau du textile et/ou sur le textile (202).

2. Procédé selon la revendication 1, dans lequel l'information de structure indique en outre la structure du matériau, le type de matériau, la répartition du matériau, l'usure du matériau du textile (202) ou une combinaison de ceux-ci.

3. Procédé selon la revendication 1 ou 2, dans lequel l'au moins un paramètre de traitement indique une recommandation d'un prétraitement, d'un traitement de nettoyage et/ou d'un traitement final du textile (202), et/ou
dans lequel l'au moins un paramètre de traitement indique un type d'agent de nettoyage, une quantité d'agent de nettoyage, une température de nettoyage, un type de dispositif de nettoyage, des réglages d'un dispositif de nettoyage (214) ou une combinaison de ceux-ci.

4. Procédé selon l'une des revendications 1 à 3, le procédé comprenant en outre :
- la mise en oeuvre ou le lancement de la mise en oeuvre d'un traitement du textile (202) conformément à l'au moins un paramètre de traitement défini par l'intermédiaire d'au moins un dispositif de traitement (222), en particulier d'un dispositif de nettoyage (214).

5. Procédé selon l'une des revendications 1 à 4, dans lequel l'information de structure est déterminée avant, pendant et/ou après un traitement du textile (202).

6. Procédé selon l'une des revendications 1 à 5, dans lequel, pour la détermination de l'information de structure du textile (202), au moins un élément formant capteur optique est utilisé.

7. Procédé selon la revendication 6, dans lequel l'élément formant capteur optique fournit une résolution spatiale tridimensionnelle.

8. Procédé selon la revendication 6 ou 7, dans lequel l'au moins un élément formant capteur optique comprend au moins un élément de type caméra (208) et fournit une information d'image du textile (202).

9. Procédé selon la revendication 8, dans lequel l'information d'image comprend au moins deux images individuelles du textile (202).

10. Procédé selon l'une des revendications 1 à 9, dans lequel l'information de structure du textile (202) comprend une information d'image d'une inscription d'un marquage du textile (202) et en particulier une reconnaissance de texte et/ou une classification de l'inscription sont effectuées.

11. Procédé selon l'une des revendications 1 à 10, dans lequel, pour la détermination de l'information de structure du textile (202), au moins un élément formant capteur acoustique est utilisé.

12. Procédé selon l'une des revendications 1 à 11, le procédé comprenant en outre :
- l'exposition du textile (202) à une excitation, en particulier l'exposition à un rayonnement et/ou à un bruit.

13. Procédé selon l'une des revendications 1 à 12, le procédé comprenant en outre :
- la détermination d'un profil utilisateur au moins en partie sur la base de l'information de structure, en particulier sur la base d'une pluralité d'informations de structure,
- dans lequel la définition de l'au moins un paramètre de traitement est basée au moins en partie sur le profil utilisateur.

14. Dispositif, dans lequel le dispositif contient un élément formant capteur optique qui est configuré pour fournir une résolution spatiale tridimensionnelle, et dans lequel le dispositif contient en outre des moyens qui sont configurés pour mettre en oeuvre et/ou commander toutes les étapes de l'un des procédés selon l'une des revendications 1 à 13.
